# EUROPEAN PATENT APPLICATION

(11) **EP 4 545 081 A1**
(43) Date of publication of application: **30.04.2025**
(21) Application number: 23843428.6
(22) Date of filing: 21.07.2023
(51) Int. Cl.: A61K 31/52, A61P 3/04, A23L 33/10

(54) **LIGAND ACTING ON ADENOSINE RECEPTORS AND COMPOSITION COMPRISING SAME FOR PREVENTION, ALLEVIATION, OR TREATMENT OF OBESITY**

(30) Priority: 22.07.2022 KR 20220091348
(71) Applicant: Future Medicine Co., Ltd., Seongnam-si, Gyeonggi-do 13449 (KR)
(72) Inventor: JEONG, Lak Shin, Seoul 06216 (KR); LEE, Yun-Hee, Seoul 08826 (KR); TRIPATHI, Sushil Kumar, Seoul 08826 (KR); KIM, Minjae, Yongin-si, Gyeonggi-do 16816 (KR); KIM, Kyungmin, Daegu 42514 (KR); IM, Hyeonyeong, Seoul 07997 (KR); KIM, Gibae, Seoul 08832 (KR)
(74) Representative: Roos, Peter
(86) International application number: PCT/KR2023/010564
(87) International publication number: WO 2024/019582

(57) **Abstract**

The present invention relates to a dual-acting ligand that acts on both A_{2A} adenosine receptor and/or A₃ adenosine receptor, and a composition including the ligand for the prevention, alleviation, or treatment of obesity. Additionally, the present invention relates to a method for treating a disease associated with A_{2A} adenosine receptor (AR) and/or A₃ adenosine receptor (AR) by using the ligand and a use of the ligand in preparing a drug for the treatment of a disease associated with A_{2A} adenosine receptor (AR) and/or A₃ adenosine receptor (AR). Serving in a dual mode as agonists for A_{2A}AR and antagonists for A_{3A}R, the ligand LJ-4378 and derivatives thereof that act on the adenosine receptors according to the present invention significantly increased mitochondrial proteins, UCP1, and COXIV and exhibited excellent anti-obesity effects in high-fat diet mice. Therefore, the ligand acting on adenosine receptors according to the present invention can be effectively used in a composition for the prevention, alleviation, or treatment of obesity, and in a therapeutic method and preparation of a drug for the same.

## Description

### CROSS-REFERENCE TO RELATED APPLICATION

The present application claims priority to and the benefit of Korean Patent Application No. 10-2022-0091348 filed in the Korean Intellectual Property Office on July 22, 2022, the entire contents of which are incorporated herein by reference.

### BACKGROUND

### 1. Field of the Invention

The present invention relates to a dual-acting ligand that acts on both A_{2A} adenosine receptor and/or A₃ adenosine receptor, and a composition including the ligand for the prevention, alleviation, or treatment of obesity.

Additionally, the present invention relates to a method for treating a disease associated with A_{2A} adenosine receptor (AR) and/or A₃ adenosine receptor (AR) by using the ligand and a use of the ligand in preparing a drug for the treatment of a disease associated with A_{2A} adenosine receptor (AR) and/or A₃ adenosine receptor (AR).

The present invention was made under the support of the Ministry of Health and Welfare of the Republic of Korea under the project number HN22C0687 (1465037037).

### 2. Discussion of Related Art

Obesity is defined as excessive fat accumulation, which is a major risk factor for metabolic diseases such as type 2 diabetes. Adipose tissue may be classified into brown adipose tissue (BAT) and white adipose tissue (WAT). While BAT contains numerous mitochondria and is responsible for non-shivering thermogenesis, WAT specializes in fat storage and mobilization. Accordingly, the activation and recruitment of BAT is a strategy to increase energy expenditure, and may ultimately be developed as a potential anti-obesity target.

As a brown adipocyte inducer, the pharmacological stimulation of adenosine receptor signaling has attracted considerable attention as a potential target for improving metabolic health. Adenosine receptors may be classified into four types of G-coupled receptors: A₁, A_{2A}, A_{2B}, and A₃. A₁ adenosine receptor (A₁AR) and A₃ adenosine receptor (A₃AR) are coupled to G_{i/o} proteins to inhibit the cAMP production and cAMP-dependent protein kinase (PKA) signaling. In contrast, A_{2A} adenosine receptor (A_{2A}AR) and A_{2B} adenosine receptor (A_{2B}AR) are coupled to G_{s/olf} proteins to facilitate adenylate cyclase (AC) activity and cAMP production.

Among adenosine receptors, the A_{2A}AR is more abundantly expressed in BAT than in WAT, and its anti-obesity effects have been supported by several studies. A_{2A}AR activation stimulates AC action to facilitate PKA-dependent lipolysis, and A_{2A}AR signaling is required for the complete physiological function of BAT. Further, recent studies have shown that an A_{2A}AR agonist CGS21680 significantly improves the expression of thermogenic genes including UCP1, both *in vitro* and *in vivo.* In contrast, the A₃AR is coupled to Gi, suggesting an inhibitory effect on A_{2A}AR stimulation. Although A₃AR expression is abundant in white adipose tissue, the regulatory roles of A₃AR in adipocyte metabolism are not yet fully understood.

### [Related Art Documents]

### [Patent Document]

KR 10-2013-0028732 (2013-03-19)

### [Non-Patent Document]

Jeong, L. S.; Lee, H. W.; Jacobson, K. A.; Kim, H. O.; Shin, D. H.; Lee, J. A.; Gao, Z.-G.; Lu, C.; Duong, H. T.; Gunaga, P.; Lee, S. K.; Jin, D. Z.; Chun, M. W.; Moon, H. R. Structureactivity relationships of 2-chloro-N6-substituted-40-thioadenosine-50-uronamides as highly potent and selective agonists at the human A3 adenosine receptor. J. Med. Chem. 2006, 49, 273-281.

### SUMMARY OF THE INVENTION

As a result of intensive efforts to provide a dual-acting ligand that acts on both A_{2A} adenosine receptor (AR) and/or A₃ adenosine receptor (AR), the present inventors have confirmed that LJ-4378 and derivatives thereof act as an agonist for the A_{2A}AR and an antagonist for the A_{3A}R, and may provide excellent anti-obesity effects, thereby completing the present invention.

Therefore, an object of the present invention is to provide a ligand that acts on an adenosine receptor, and a composition including the ligand for the prevention, alleviation, or treatment of obesity.

Another object of the present invention is to provide a method for treating a disease associated with A_{2A} adenosine receptor (AR) and/or A₃ adenosine receptor (AR) by using the ligand and a use of the ligand in preparing a drug for the treatment of a disease associated with A_{2A} adenosine receptor (AR) and/or A₃ adenosine receptor (AR).

The present invention provides a pharmaceutical composition for the prevention or treatment of a disease associated with A_{2A} adenosine receptor (AR) and/or A₃ adenosine receptor (AR), including a compound represented by the following [Chemical Formula 1], an isomer thereof, or a pharmaceutically acceptable salt thereof: 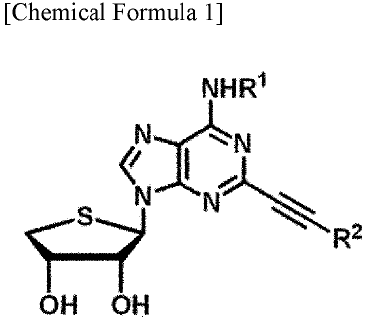 wherein R1 is H, a C1-C10 alkyl group, or an aryl-C1-C10 alkyl group, and
R2 is a C1-C10 alkyl group.

According to a preferred embodiment of the present invention, R1 is H, a methyl group, or an aryl-C1-C10 alkyl group, and
R2 is a C1-C4 alkyl group.

According to a preferred embodiment of the present invention, the compound represented by [Chemical Formula 1], the isomer thereof, or the pharmaceutically acceptable salt thereof is any one or more selected from the group consisting of
(1) 2-(6-amino-2-(hex-1-yn-1-yl)-9H-purin-9-yl)tetrahydrothiophene-3,4-diol;
(2) (2R,3R,4S)-2-(6-amino-2-(prop-1-yn-1-yl)-9H-purin-9-yl)tetrahydrothiophene-3,4-diol;
(3) (2R,3R,4S)-2-(6-((3-fluorobenzyl)amino)-2-(prop-1-yn-1-yl)-9H-purin-9-yl)tetrahydrothiophene-3,4-diol;
(4) (2R,3R,4S)-2-(6-((3-chlorobenzyl)amino)-2-(prop-1-yn-1-yl)-9H-purin-9-yl)tetrahydrothiophene-3,4-diol;
(5) (2R,3R,4S)-2-(6-((3-bromobenzyl)amino)-2-(prop-1-yn-1-yl)-9H-purin-9-yl)tetrahydrothiophene-3,4-diol;
(6) (2R,3R,4S)-2-(6-((3-iodobenzyl)amino)-2-(prop-1-yn-1-yl)-9H-purin-9-yl)tetrahydrothiophene-3,4-diol;
(7) (2R,3R,4S)-2-(6-amino-2-(but-1-yn-1-yl)-9H-purin-9-yl)tetrahydrothiophene-3,4-diol;
(8) (2R,3R,4S)-2-(2-(but-1-yn-1-yl)-6-((3-fluorobenzyl)amino)-9H-purin-9-yl)tetrahydrothiophene-3,4-diol;
(9) (2R,3R,4S)-2-(2-(but-1-yn-1-yl)-6-((3-chlorobenzyl)amino)-9H-purin-9-yl)tetrahydrothiophene-3,4-diol;
(10) (2R,3R,4S)-2-(6-((3-bromobenzyl)amino)-2-(but-1-yn-1-yl)-9H-purin-9-yl)tetrahydrothiophene-3,4-diol; and
(11) (2R,3R,4S)-2-(2-(but-1-yn-1-yl)-6-((3-iodobenzyl)amino)-9H-purin-9-yl)tetrahydrothiophene-3,4-diol.

According to a preferred embodiment of the present invention, the compound represented by [Chemical Formula 1], the isomer thereof, or the pharmaceutically acceptable salt thereof is an agonist for A_{2A} adenosine receptor (AR) and an antagonist for A₃ adenosine receptor (AR).

According to a preferred embodiment of the present invention, the disease associated with A_{2A} adenosine receptor (AR) and/or A₃ adenosine receptor (AR) is obesity.

Further, the present invention provides a health functional food composition for the prevention or alleviation of obesity, including a compound represented by the following [Chemical Formula 1], an isomer thereof, or a pharmaceutically acceptable salt thereof: 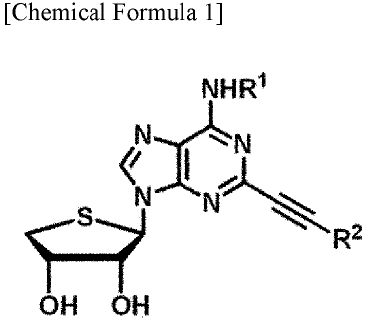 wherein R1 is H, a C1-C10 alkyl group, or an aryl-C1-C10 alkyl group, and
R2 is a C1-C10 alkyl group.

In addition, the present invention provides a method for treating a disease associated with A_{2A} adenosine receptor (AR) and/or A₃ adenosine receptor (AR), the method including administering a compound represented by the following [Chemical Formula 1], an isomer thereof, or a pharmaceutically acceptable salt thereof to a patient with a disease associated with A_{2A} adenosine receptor (AR) and/or A₃ adenosine receptor (AR): 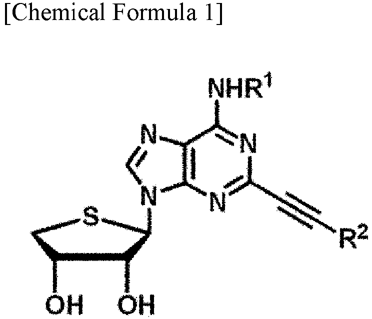 wherein R1 is H, a C1-C10 alkyl group, or an aryl-C1-C10 alkyl group, and
R2 is a C1-C10 alkyl group.

Furthermore, the present invention provides a use of a compound represented by the following [Chemical Formula 1], an isomer thereof, or a pharmaceutically acceptable salt thereof in preparing a drug for the treatment of a disease associated with A_{2A} adenosine receptor (AR) and/or A₃ adenosine receptor (AR): 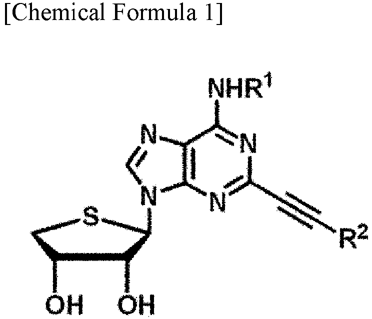 wherein R1 is H, a C1-C10 alkyl group, or an aryl-C1-C10 alkyl group, and
R2 is a C1-C10 alkyl group.

Extracellular adenosine is a potent endogenous signaling molecule that regulates multiple physiological and pathological events via four types of adenosine receptors. Studies using transgenic mice with altered adenosine receptor expression further support that adenosine exerts various effects on energy metabolism. Accordingly, targeting adenosine receptors may have translational potential for the treatment of metabolic diseases. In the present invention, the effects of a dual-acting ligand with A_{2A}AR agonist and A₃AR antagonist activities on adipocyte metabolism were confirmed.

While the stimulation of G_{αs}-coupled A_{2A} adenosine receptor activates adenylate cyclase and cAMP-PKA signaling pathways subsequently, the activation of G_{αi}-coupled A₃AR inhibits adenylate cyclase, thereby opposing the effects of A_{2A}AR signaling. Accordingly, the present inventors hypothesized that the dual ligand with A_{2A}AR agonist and A₃AR antagonist activities could synergistically activate PKA-dependent lipolysis and thermogenesis in adipocytes.

In the present invention, it was confirmed that LJ-4378 treatment increased the mitochondrial content and activity in adipocytes, and the effects of LJ-4378 were more potent than those of the A_{2A}AR agonist or A₃AR antagonist. *In vivo* LJ-4378 treatment increased energy expenditure and the expression of a brown adipocyte marker CIDEA, which were determined by non-invasive imaging of bioluminescence signaling in CIDEA reporter mice. *In vivo* LJ-4378 treatment reduced body weight and fat content and improved glucose tolerance in HFD-fed mice, thereby indicating its anti-obesity effects.

In the present invention, qPCR analysis indicated that A_{2A}AR and A₃AR are the major subtypes of adenosine receptors expressed in adipocytes. In contrast, the expression levels of A_{2B}AR in adipocytes were lower than those of other adenosine receptors. Previous studies demonstrated that A_{2B}AR is expressed mainly in the muscle and BAT, and the activation of A_{2B}AR exerts anti-aging and anti-obesity effects. Interestingly, A_{2B}AR plays a permissive role in A_{2A}-mediated lipolysis to form heterodimers of the two receptors. In contrast, A₁ receptors, which are coupled to G_{αi}, inhibit adenylate cyclase activity and are highly expressed in human white adipocytes. Accordingly, A₁ARs have been characterized as lipolysis inhibitors capable of promoting insulin sensitivity in adipose tissue by reducing circulating FFA and triglyceride levels. Moreover, A₁AR signaling promotes lipogenesis and modulates inflammation, as shown in an A₁AR knockout mouse study.

In addition to its effects on lipolysis, A_{2A}AR activation exerts anti-inflammatory effects in a mouse model of diet-induced obesity.

In conclusion, the present invention can be administered orally, and thus is highly favorable to patients, and since its mechanism of action is not to stimulate the central nervous system but to promote the breakdown and consumption of fat via the adenosine receptors in adipocytes, it is less likely for side effect problems in the psychiatric system to occur. As an anti-obesity drug with a new mechanism, it is possible to provide an option for patients who do not respond to existing therapeutic methods.

Furthermore, while existing anti-obesity drugs consisting of GLP-1 derivatives and central nervous system stimulants treat obesity due to an effect of reducing appetite, the present invention may increase the preference of patients through the mechanism of reducing fat by directly targeting brown adipocytes, and can be expected to enhance effects and mitigate side effects through combined administration.

Therefore, the present invention may provide a pharmaceutical composition for the prevention or treatment of a disease associated with A_{2A} adenosine receptor (AR) and/or A₃ adenosine receptor (AR), including a compound represented by the following [Chemical Formula 1], an isomer thereof, or a pharmaceutically acceptable salt thereof: 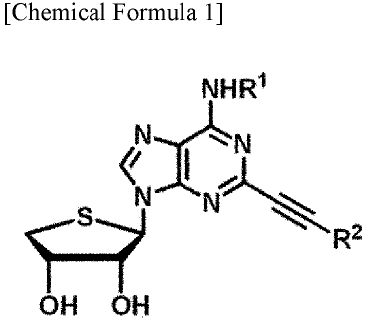 wherein R1 is H, a C1-C10 alkyl group, or an aryl-C1-C10 alkyl group, and
R2 is a C1-C10 alkyl group.

According to a preferred embodiment of the present invention, R1 is H, a methyl group, or an aryl-C1-C10 alkyl group, and
R2 may be a C1-C4 alkyl group.

In the present invention, an acid addition salt formed by a pharmaceutically acceptable free acid is useful as the pharmaceutically acceptable salt. The acid addition salt is obtained from inorganic acids such as hydrochloric acid, nitric acid, phosphoric acid, sulfuric acid, hydrobromic acid, hydroiodic acid, nitrous acid or phosphorous acid, and nontoxic organic acids such as aliphatic mono- and dicarboxylates, phenyl-substituted alkanoates, hydroxyalkanoates and alkanedioates, aromatic acids, aliphatic and aromatic sulfonic acids. Such pharmaceutically nontoxic salts include sulfate, pyrosulfate, bisulfate, sulfite, bisulfite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methyl benzoate, dinitrobenzoate, hydroxybenzoate, methoxybenzoate, phthalate, terephthalate, benzenesulfonate, toluenesulfonate, chlorobenzenesulfonate, xylenesulfonate, phenyl acetate, phenyl propionate, phenyl butyrate, citrate, lactate, βhydroxybutyrate, glycolate, malate, tartrate, methanesulfonate, propanesulfonate, naphthalene-1-sulfonate, naphthalene-2-sulfonate, or mandelate.

The acid addition salt according to the present invention may be prepared by typical methods, for example, dissolving a compound represented by [Chemical Formula 1] in an excess aqueous acid solution, and precipitating this salt using a water-miscible organic solvent, for example, methanol, ethanol, acetone or acetonitrile. Further, the acid addition salt may also be prepared by evaporating the solvent or excess acid from this mixture, and then drying the mixture or suction-filtering a precipitated salt.

In addition, a pharmaceutically acceptable metal salt may be prepared using a base. An alkali metal or alkaline earth metal salt is obtained by, for example, dissolving the compound in an excess alkali metal hydroxide or alkaline-earth metal hydroxide solution, filtering the non-soluble compound salt, evaporating the filtrate, and drying the resulting product. In this case, preparing a sodium, potassium or calcium salt as the metal salt is pharmaceutically suitable. A silver salt corresponding to this is obtained by reacting the alkali metal or alkaline earth metal salt with a suitable silver salt (for example, silver nitrate).

According to a preferred embodiment of the present invention, the compound represented by [Chemical Formula 1], the isomer thereof, or the pharmaceutically acceptable salt thereof may be any one or more selected from the group consisting of
(1) 2-(6-amino-2-(hex-1-yn-1-yl)-9H-purin-9-yl)tetrahydrothiophene-3,4-diol;
(2) (2R,3R,4S)-2-(6-amino-2-(prop-1-yn-1-yl)-9H-purin-9-yl)tetrahydrothiophene-3,4-diol;
(3) (2R,3R,4S)-2-(6-((3-fluorobenzyl)amino)-2-(prop-1-yn-1-yl)-9H-purin-9-yl)tetrahydrothiophene-3,4-diol;
(4) (2R,3R,4S)-2-(6-((3-chlorobenzyl)amino)-2-(prop-1-yn-1-yl)-9H-purin-9-yl)tetrahydrothiophene-3,4-diol;
(5) (2R,3R,4S)-2-(6-((3-bromobenzyl)amino)-2-(prop-1-yn-1-yl)-9H-purin-9-yl)tetrahydrothiophene-3,4-diol;
(6) (2R,3R,4S)-2-(6-((3-iodobenzyl)amino)-2-(prop-1-yn-1-yl)-9H-purin-9-yl)tetrahydrothiophene-3,4-diol;
(7) (2R,3R,4S)-2-(6-amino-2-(but-1-yn-1-yl)-9H-purin-9-yl)tetrahydrothiophene-3,4-diol;
(8) (2R,3R,4S)-2-(2-(but-1-yn-1-yl)-6-((3-fluorobenzyl)amino)-9H-purin-9-yl)tetrahydrothiophene-3,4-diol;
(9) (2R,3R,4S)-2-(2-(but-1-yn-1-yl)-6-((3-chlorobenzyl)amino)-9H-purin-9-yl)tetrahydrothiophene-3,4-diol;
(10) (2R,3R,4S)-2-(6-((3-bromobenzyl)amino)-2-(but-1-yn-1-yl)-9H-purin-9-yl)tetrahydrothiophene-3,4-diol; and
(11) (2R,3R,4S)-2-(2-(but-1-yn-1-yl)-6-((3-iodobenzyl)amino)-9H-purin-9-yl)tetrahydrothiophene-3,4-diol.

According to a preferred embodiment of the present invention, the compound represented by [Chemical Formula 1], the isomer thereof, or the pharmaceutically acceptable salt thereof may be an agonist for A_{2A} adenosine receptor (AR) and an antagonist for A₃ adenosine receptor (AR).

According to a preferred embodiment of the present invention, the disease associated with A_{2A} adenosine receptor (AR) and/or A₃ adenosine receptor (AR) may be obesity.

The 'prevention' of the present invention refers to all actions that suppress a disease associated with A_{2A} adenosine receptor (AR) and/or A₃ adenosine receptor (AR) or delay the onset thereof due to the compound represented by [Chemical Formula 1] of the present invention, the isomer thereof, or the pharmaceutically acceptable salt thereof.

The 'alleviation' or 'treatment' of the present invention refers to all actions that ameliorate or benefit parameters, such as the severity of symptoms, associated with a disease associated with A_{2A} adenosine receptor (AR) and/or A₃ adenosine receptor (AR) due to the compound represented by [Chemical Formula 1] of the present invention, the isomer thereof, or the pharmaceutically acceptable salt thereof.

The pharmaceutical composition of the present invention may be in the form of various oral or parenteral formulations. When the composition is formulated, the composition may be prepared by using a buffer (for example, a saline solution or PBS), an antioxidant, a bacteriostatic agent, a chelating agent (for example, EDTA or glutathione), a filler, an extender, a binder, an adjuvant (for example, aluminum hydroxide), a suspension agent, a thickener, a wetting agent, a disintegrant, or a surfactant, a diluent or an excipient.

Examples of a solid preparation for oral administration include a tablet, a pill, a powder, granules, a capsule, and the like, and the solid preparation is prepared by mixing one or more compounds with one or more excipients, for example, starch (including corn starch, wheat starch, rice starch, potato starch, and the like), calcium carbonate, sucrose, lactose, dextrose, sorbitol, mannitol, xylitol, erythritol maltitol, cellulose, methyl cellulose, sodium carboxymethylcellulose and hydroxypropyl methyl-cellulose, gelatin, or the like. For example, a tablet or a sugar-coated tablet may be obtained by blending an active ingredient with a solid excipient, pulverizing the resulting blend, adding a suitable auxiliary agent thereto, and then processing the resulting mixture into a granular mixture.

Further, in addition to simple excipients, lubricants such as magnesium stearate and talc are also used. A liquid preparation for oral administration corresponds to a suspension agent, a liquid for internal use, an emulsion, a syrup, and the like, and the liquid preparation may include, in addition to water and liquid paraffin which are simple commonly used diluents, various excipients, for example, a wetting agent, a sweetener, an odorant, a preservative, and the like. In addition, in some cases, cross-linked polyvinyl pyrrolidone, agar, alginic acid, sodium alginate, or the like may be added as a disintegrant, and an anticoagulant, a fragrance, an emulsifier, a solubilizer, a dispersant, a flavoring agent, an antioxidant, a packaging agent, a pigment, a preservative, and the like may be additionally included.

Examples of a preparation for parenteral administration include an aqueous sterile solution, a non-aqueous solvent, a suspension solvent, an emulsion, a freeze-dried preparation, a suppository, or the like. As the non-aqueous solvent and the suspension solvent, it is possible to use propylene glycol, polyethylene glycol, a vegetable oil such as olive oil, an injectable ester such as ethyl oleate, and the like. As a base of the suppository, it is possible to use Witepsol, Macrogol, Tween 61, cacao butter, laurinum, glycerol, gelatin, and the like.

The composition of the present invention may be administered orally or parenterally, and, when administered parenterally, may be formulated in the form of a preparation for external application to the skin; an injection administered intraperitoneally, rectally, intravenously, subcutaneously, or a percutaneous administration agent according to a method known in the art.

The injection must be sterilized and protected from contamination of microorganisms such as bacteria and fungi. Examples of a suitable carrier for the injection may be, but are not limited to, a solvent or a dispersion medium including water, ethanol, polyols (for example, glycerol, propylene glycol, liquid polyethylene glycol, and the like), mixtures thereof, and/or vegetable oils. More preferably, as a suitable carrier, it is possible to use an isotonic solution such as Hank's solution, Ringer's solution, triethanolamine-containing phosphate buffered saline (PBS) or sterile water for injection, 10% ethanol, 40% propylene glycol, and 5% dextrose, and the like. To protect the injection from microbial contamination, various antimicrobial agents and antifungal agents such as a paraben, chlorobutanol, phenol, sorbic acid, and thimerosal may be additionally included. Furthermore, in most cases, the injection may additionally include an isotonic agent such as sugar or sodium chloride.

Examples of the percutaneous administration agent include a form such as an ointment, a cream, a lotion, a gel, a solution for external use, a paste, a liniment, and an aerosol. The transdermal administration as described above means that an effective amount of an active ingredient contained in a pharmaceutical composition is delivered into the skin via local administration thereof to the skin.

The composition of the present invention is administered in a pharmaceutically effective amount. The pharmaceutically effective amount means an amount sufficient to treat diseases at a reasonable benefit/risk ratio applicable to medical treatment, and an effective dosage level may be determined according to factors including the type of diseases of patients, the severity of disease, the activity of drugs, sensitivity to drugs, administration time, administration route, excretion rate, treatment period, and simultaneously used drugs, and other factors well known in the medical field. The composition of the present invention may be administered as an individual therapeutic agent or in combination with other therapeutic agents, may be administered sequentially or simultaneously with therapeutic agents in the related art, and may be administered in a single dose or multiple doses. That is, the total effective amount of the composition of the present invention may be administered to a patient in a single dose or may be administered by a fractionated treatment protocol, in which multiple doses are administered over a long period of time. It is important to administer the composition in a minimum amount that can obtain the maximum effect without any side effects, in consideration of all the aforementioned factors, and this may be easily determined by a person skilled in the art.

A dosage of the pharmaceutical composition of the present invention may vary according to body weight, age, gender, and health status of a patient, age of a patient, diet, administration time, administration method, excretion rate, and the severity of a disease.

The composition of the present invention may be used either alone or in combination with surgery, radiation therapy, hormone therapy, chemotherapy, and methods using a biological response modifier.

The pharmaceutical composition of the present invention may also be provided in the form of a formulation of an external preparation including the compound represented by Chemical Formula 1, the isomer thereof or the pharmaceutically acceptable salt thereof as an active ingredient. When the pharmaceutical composition for the prevention or treatment of a disease associated with A_{2A} adenosine receptor (AR) and/or A₃ adenosine receptor (AR) according to the present invention is used as a preparation for external application to the skin, the pharmaceutical composition may additionally contain auxiliary agents typically used in the dermatology field, such as any other ingredients typically used in the preparation for external application to the skin, such as a fatty substance, an organic solvent, a solubilizing agent, a thickener and a gelling agent, a softener, an antioxidant, a suspending agent, a stabilizer, a foaming agent, an odorant, a surfactant, water, an ionic emulsifier, a non-ionic emulsifier, a filler, a metal ion blocking agent, a chelating agent, a preservative, a vitamin, a blocking agent, a wetting agent, an essential oil, a dye, a pigment, a hydrophilic active agent, a lipophilic active agent, or a lipid vesicle. In addition, the ingredients may be introduced in an amount generally used in the dermatology field.

When the pharmaceutical composition for the prevention and treatment of a disease associated with A_{2A} adenosine receptor (AR) and/or A₃ adenosine receptor (AR) according to the present invention is provided as a preparation for external application to the skin, the pharmaceutical composition may be in the form of a formulation such as an ointment, a patch, a gel, a cream, or an aerosol, but is not limited thereto.

Further, the present invention may provide a health functional food composition for the prevention or alleviation of obesity, including a compound represented by the following [Chemical Formula 1], an isomer thereof, or a pharmaceutically acceptable salt thereof: 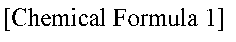 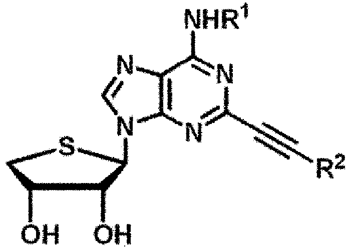 wherein R1 is H, a C1-C10 alkyl group, or an aryl-C1-C10 alkyl group, and
R2 is a C1-C10 alkyl group.

Since the compound represented by [Chemical Formula 1], the isomer thereof, or the pharmaceutically acceptable salt thereof is the same as that used in the pharmaceutical composition, the description thereof is replaced with the foregoing description.

The health functional food composition according to the present invention can be prepared in various forms by typical methods known in the art. A general food can be prepared by adding the compound represented by [Chemical Formula 1] of the present invention, the isomer thereof, or the pharmaceutically acceptable salt thereof to, without being limited to, a beverage (including an alcoholic beverage), fruit and a processed food thereof (for example: canned fruit, bottled food, jam, marmalade, and the like), fish, meat and processed food thereof (for example: ham, sausage, corned beef, and the like), bread and noodles (for example: thick wheat noodles, buckwheat noodles, instant noodles, spaghetti, macaroni, and the like), fruit juice, various drinks, cookies, wheat-gluten, dairy products (for example: butter, cheese, and the like), edible vegetable oils, margarine, vegetable protein, retort foods, frozen food and various seasonings (for example: soybean paste, soy sauce, sauce, and the like), and the like. In addition, a nutritional supplement may be prepared by adding the compound represented by [Chemical Formula 1] of the present invention, the isomer thereof or the pharmaceutically acceptable salt thereof to, but not limited to, a capsule, a tablet, a pill, and the like. Furthermore, for a health functional food, for example, the compound represented by [Chemical Formula 1] of the present invention, the isomer thereof, or the pharmaceutically acceptable salt thereof itself is prepared in the form of, without being limited to, tea, juice, and drink and can be taken by being processed into a liquid, a granule, a capsule, and a powder so as to be able to be drunk (health beverage). Further, in order to use the compound represented by [Chemical Formula 1] of the present invention, the isomer thereof, or the pharmaceutically acceptable salt thereof in the form of a food additive, it may be prepared in the form of a powder or a concentrated liquid and used. In addition, the compound represented by [Chemical Formula 1] of the present invention, the isomer thereof, or the pharmaceutically acceptable salt thereof may be mixed with a known active ingredient known to have an effect of preventing or alleviating a disease associated with A_{2A} adenosine receptor (AR) and/or A₃ adenosine receptor (AR) to be prepared in the form of a composition.

When the compound represented by [Chemical Formula 1] of the present invention, the isomer thereof, or the pharmaceutically acceptable salt thereof is used as a health beverage, the health beverage composition may contain various flavoring agents or natural carbohydrates, and the like, such as typical beverages, as additional ingredients. The above-described natural carbohydrates may be monosaccharides such as glucose and fructose; disaccharides such as maltose and sucrose; polysaccharides such as dextrin and cyclodextrin; and sugar alcohols such as xylitol, sorbitol, and erythritol. As a sweetener, it is possible to use a natural sweetener such as thaumatin and stevia extract; a synthetic sweetener such as saccharin and aspartame, and the like.

Furthermore, the compound represented by [Chemical Formula 1] of the present invention, the isomer thereof, or the pharmaceutically acceptable salt thereof may be contained as an active ingredient in a health functional food composition for the prevention or alleviation of a disease associated with A_{2A} adenosine receptor (AR) and/or A₃ adenosine receptor (AR), and the amount of the active ingredient is not particularly limited to an amount effective for achieving the action of preventing or alleviating a disease associated with A_{2A} adenosine receptor (AR) and/or A₃ adenosine receptor, but is preferably 0.01 to 100 wt% based on the total weight of the entire composition. The health functional food composition of the present invention may be prepared by mixing the compound represented by [Chemical Formula 1], the isomer thereof, or the pharmaceutically acceptable salt thereof with other active ingredients known to be effective against a disease associated with A_{2A} adenosine receptor (AR) and/or A₃ adenosine receptor (AR).

In addition to the aforementioned ingredients, the health functional food of the present invention may contain various nutrients, vitamins, electrolytes, flavoring agents, coloring agents, pectic acid, salts of pectic acid, alginic acid, salts of alginic acid, organic acids, protective colloidal thickeners, pH adjusting agents, stabilizers, preservatives, glycerin, alcohols, carbonate agents, and the like. Further, the health functional food of the present invention may contain a flesh for preparing natural fruit juice, fruit juice beverage, or vegetable beverage. These ingredients may be used either alone or in mixtures thereof.

In addition, the present invention may provide a method for treating a disease associated with A_{2A} adenosine receptor (AR) and/or A₃ adenosine receptor (AR), the method including administering a compound represented by the following [Chemical Formula 1], an isomer thereof, or a pharmaceutically acceptable salt thereof to a patient with a disease associated with A_{2A} adenosine receptor (AR) and/or A₃ adenosine receptor (AR): 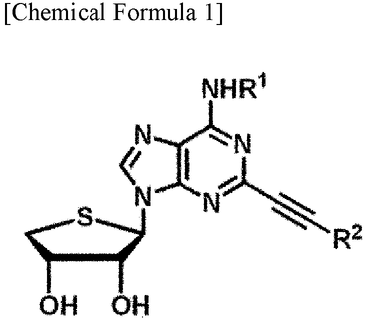 wherein R1 is H, a C1-C10 alkyl group, or an aryl-C1-C10 alkyl group, and
R2 is a C1-C10 alkyl group.

Since the compound represented by [Chemical Formula 1], the isomer thereof, or the pharmaceutically acceptable salt thereof is the same as that used in the pharmaceutical composition, the description thereof is replaced with the foregoing description.

According to a preferred embodiment of the present invention, the disease associated with A_{2A} adenosine receptor (AR) and/or A₃ adenosine receptor (AR) may be obesity.

Furthermore, the present invention may provide a use of a compound represented by the following [Chemical Formula 1], an isomer thereof, or a pharmaceutically acceptable salt thereof in preparing a drug for the treatment of a disease associated with A_{2A} adenosine receptor (AR) and/or A₃ adenosine receptor (AR): 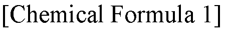 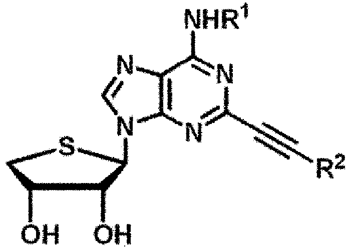 wherein R1 is H, a C1-C10 alkyl group, or an aryl-C1-C10 alkyl group, and
R2 is a C1-C10 alkyl group.

Since the compound represented by [Chemical Formula 1], the isomer thereof, or the pharmaceutically acceptable salt thereof is the same as that used in the pharmaceutical composition, the description thereof is replaced with the foregoing description.

According to a preferred embodiment of the present invention, the disease associated with A_{2A} adenosine receptor (AR) and/or A₃ adenosine receptor (AR) may be obesity.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:

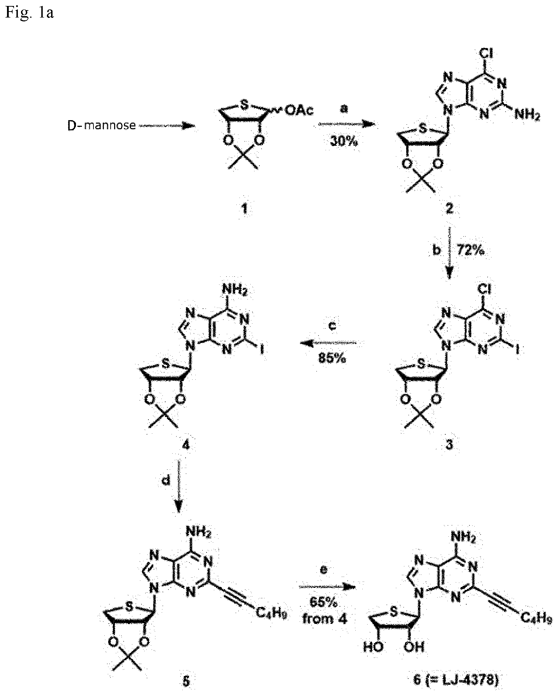

FIG. 1A shows the synthesis process of LJ-4378 (compound 6)(a: Silylated 2-amino-6-chloropurine, TMSOTf, DCE, room temperature to 80°C 3 h; b: CuI, isoamyl nitrite, I₂, CH₂I₂, THF, 110°C 45 min; c: NH₃/MeOH, 80°C 2 h; d: 1-Hexyne, CuI, TEA, DMF, bis(triphenylphosphine)palladium dichloride, room temperature, 3 h; e: 1 N HCl, THF, room temperature, 15 h).

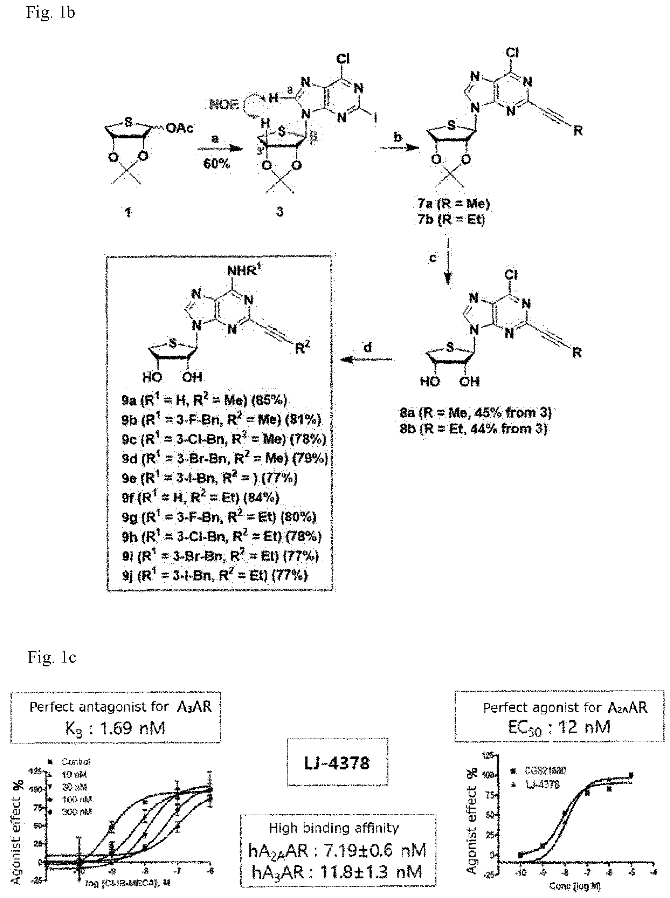

FIG. 1B shows the synthesis process of LJ-4378 derivatives (compounds 9a to 9j)(a: 2-iodo-6-chloropurine, BSA, TMSOTf, CH₃CN, rt to 80°C 3h; b: propyne/butyne, CuI, Cs₂CO₃, Pd(PPh₃)₄, DMF, rt, 70 min; c: 1 N HCl, THF, rt, 15h; d: NH₃/tBuOH, 100°C 12h or R₁NH₂, Et₃N, EtOH, rt, 24-48h).
FIG. 1C shows the drug profile of LJ-4378.

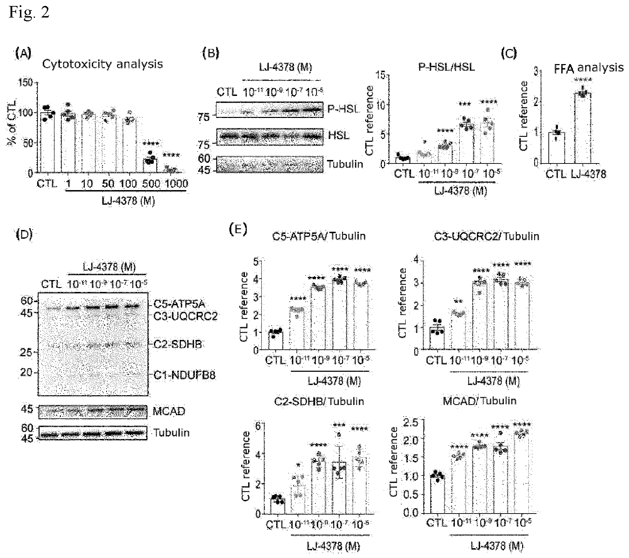

FIG. 2 shows that LJ-4378 treatment increases the lipolysis and mitochondrial content of adipocytes. (A) Evaluation of cytotoxicity of brown adipocytes (BAs) treated with LJ-4378 at the indicated concentrations for 24 hours (n = 5, mean ± SEM, **** p < 0.0001). (B) Immunoblot analysis of hormone-sensitive lipase (HSL) and phosphorylated HSL (p-HSL, serine 660) of BAs treated with LJ-4378 for 4 hours (n = 5, mean ± SEM, * p < 0.05, *** p < 0.001, **** p < 0.0001). (C) Free fatty acid (FFA) levels in conditioned medium of BAs treated with 0.1 µM LJ-4378 for 24 hours (n = 5, mean ± SEM, **** p <0.0001). (D) Immunoblot analysis of BAs treated with LJ-4378 at the indicated concentrations for 48 hours (n = 5, mean ± SEM, * p < 0.05, ** p < 0.01, *** p < 0.001, **** p < 0.0001). (E) Mitochondrial protein levels after treatment with LJ-4378 at the indicated concentrations for 48 hours (n = 5, mean ± SEM, * p < 0.05, *** p < 0.001, **** p < 0.0001).

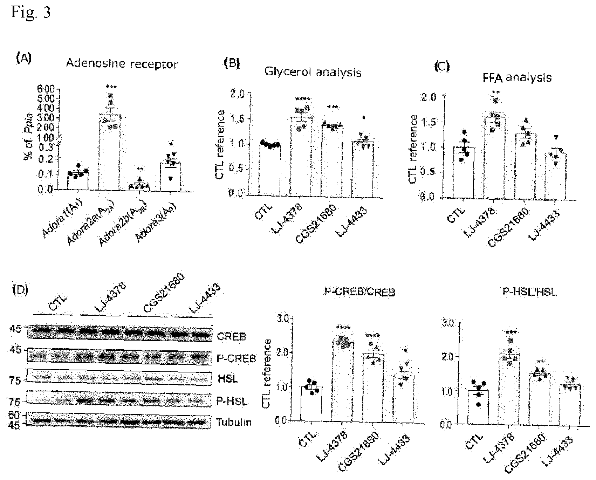

FIG. 3 shows that the lipolytic effects of LJ-4378 are more potent than those of A_{2A} agonist or A₃ antagonist. (A) qPCR analysis of expression levels of A₁, A_{2A}, A_{2B}, and A₃ adenosine receptors in brown adipocytes (BAs) (n = 5, mean ± SEM, * p < 0.05, ** p < 0.01, *** p < 0.001). (B-C) Glycerol and FFAlevels in conditioned medium of BAs treated with LJ-4378 (0.1 µM), A_{2A} antagonist (CGS21680, 0.1 µM), or A₃ antagonist (LJ-4433, 0.1 µM) for 24 hours (n = 5, mean ± SEM, * p < 0.05, ** p < 0.01, *** p < 0.001, **** p < 0.0001). (D) Immunoblot analysis of BAs treated with LJ-4378 (0.1 µM), A_{2A} antagonist (CGS21680, 0.1 µM), or A₃ antagonist (LJ-4433, 0.1 µM) for 24 hours. (n = 5, mean ± SEM, * p < 0.05, ** p < 0.01, *** p < 0.001, **** p < 0.0001).

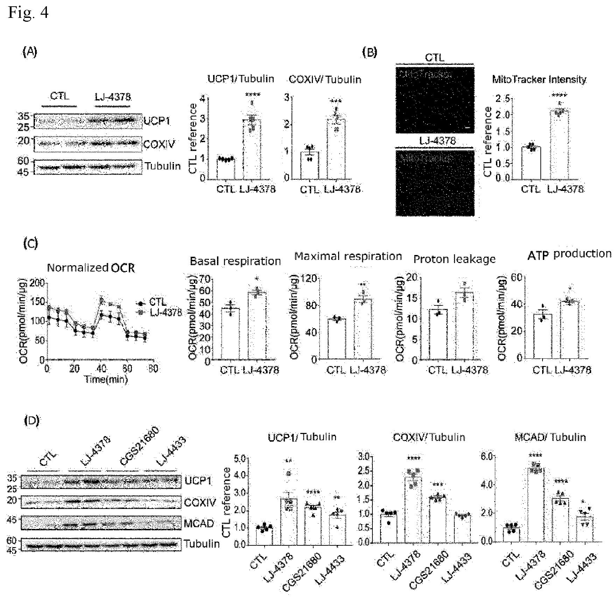

FIG. 4 shows that *in vitro* LJ-4378 treatment increased mitochondrial content and activity in brown adipocytes. (A) Immunoblot analysis of BAs treated with 0.1 µM LJ-4378 for 24 hours (n=5, mean ± SEM, ***p<0.001, ****p<0.0001). (B) Representative images of MitoTracker staining of BAs treated with vehicle (CTL) and 0.1 µM LJ-4378 (n = 5, size bar = 20 µm). (C) OCR measurements of BAs treated with 0.1 µM LJ-4378 for 24 hours (n = 3, mean ± SEM, * p < 0.05, ** p < 0.01). (D) Immunoblot analysis of BAs treated with LJ-4378 (0.1 µM), A_{2A} antagonist (CGS21680, 0.1 µM), or A₃ antagonist (LJ-4433, 0.1 µM) for 24 hours (n = 5, mean ± SEM, * p < 0.05, ** p < 0.01, *** p < 0.001, **** p < 0.0001).

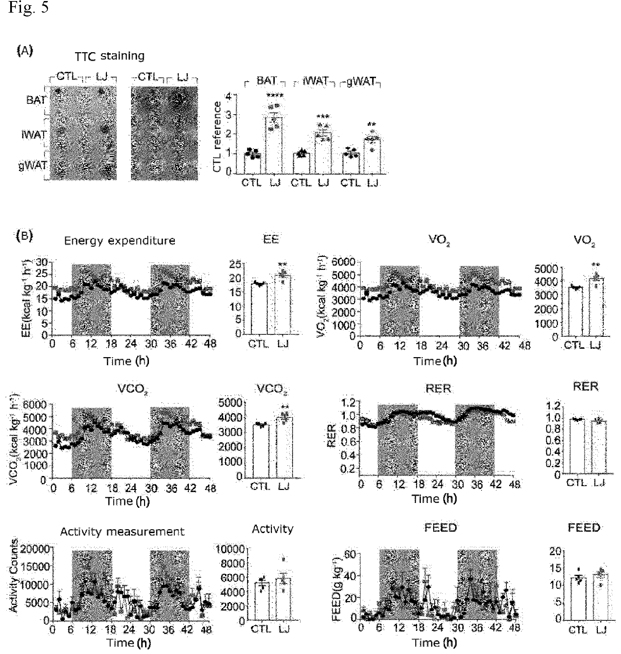

FIG. 5 shows that *in vivo* LJ-4378 treatment increased mitochondrial activity and energy expenditure of adipose tissue. (A) 2,3,5-Triphenyltetrazolium chloride (TTC) staining of BAT, iWAT, and gWAT (n = 5, mean ± SEM, ** p < 0.01, *** p < 0.001, **** p < 0.0001). (B) Indirect calorimetry of energy expenditure (EE), VO₂ (proportion of oxygen consumption), VCO₂ (proportion of carbon dioxide production), respiratory exchange ratio (RER), activity counts, and food consumption (FEED) (n = 5, mean ± SEM, ** p < 0.01).

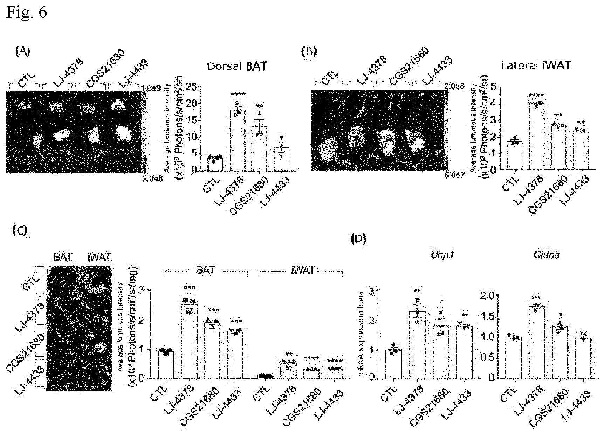

FIG. 6 shows the browning effects of LJ-4378 according to research on CIDEA reporter mice. Mice were treated with vehicle (CTL), LJ-4378 (LJ, 1 mg·kg-1/day), A_{2A} antagonist (CGS21680, 1 mg·kg-1/day), or A₃ antagonist (LJ-4433, 1 mg·kg-1/day) for 10 days. (A-B) Representative bioluminescence in dorsal and lateral positions (n = 3, mean ± SEM, ** p < 0.01, **** p < 0.0001). (C) Representative *ex vivo* fluorescence image and quantification of fluorescence intensity of BAT and iWAT (n = 3, mean ± SEM, ** p < 0.01, **** p < 0.0001). (D) qPCR analysis of UCP1 and CIDEAM RNA expression levels of brown adipose tissue (BAT) (n = 3, mean ± SEM, * p < 0.05, ** p < 0.01, *** p < 0.001).

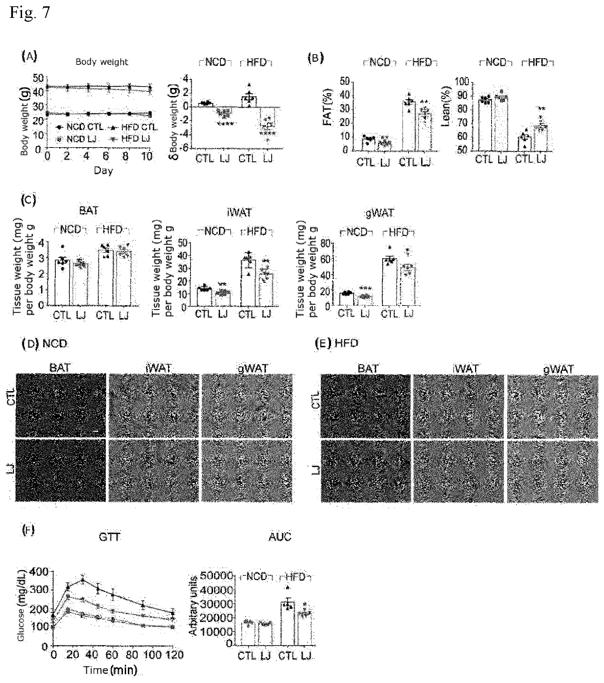

FIG. 7 shows that *in vivo* LJ-4378 treatment protected mice from high-fat diet-induced obesity. Mice fed a normal chow diet (NCD) and a high-fat diet (HFD) were treated with LJ-4378 (LJ, 1 mg·kg-1/day) or vehicle control (CTL) for 10 days. (A) Change in body weight (n = 6). (B) Body composition of percentage fat and percentage lean (n = 6, mean ± SEM, ** p < 0.01). (C) Tissue weight (mg) per g body weight for BAT, iWAT, and gWAT (n = 6, mean ± SEM, * p < 0.05, ** p < 0.01). (D-E) Hematoxylin and eosin (H/E) staining of paraffin sections of BAT, iWAT and gWAT (size bar = 10 µm). (F) Glucose tolerance test and area under the curve (AUC) (n = 5, mean ± SEM, * p < 0.05).

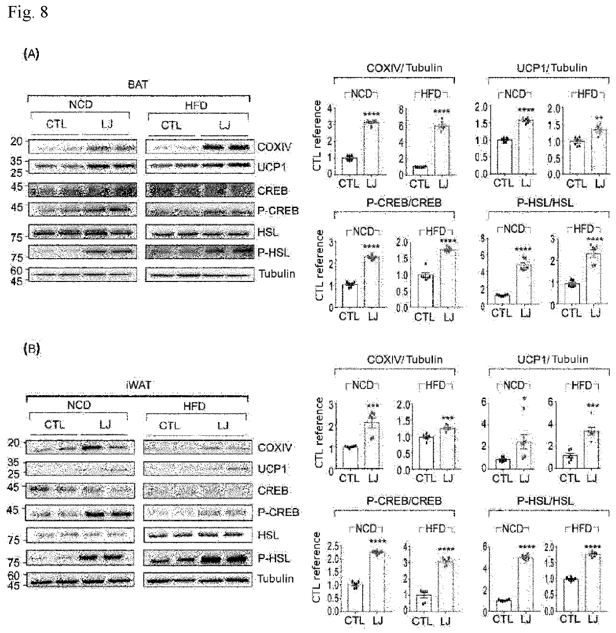

FIG. 8 shows that the anti-obesity effects of LJ-4378 involve activation of the PKA signaling pathway in adipose tissue. Mice fed a normal chow diet (NCD) and a high-fat diet (HFD) were treated with LJ-4378 (LJ, 1 mg·kg-1/day) for 10 days. (A) Immunoblot analysis of BAT (n = 6, mean ± SEM, ** p < 0.01, **** p < 0.0001). (B) Immunoblot analysis in iWAT. Tubulin was a loading control. (n = 6, mean ± SEM, * p < 0.05, *** p < 0.001, **** p < 0.0001).

### DETAILED DESCRIPTION OF EXEMPLARY EMBODIMENTS

Hereinafter, the present invention will be described in more detail through Examples. These Examples are only for exemplifying the present invention, and it is obvious to a person of ordinary skill in the art that the scope of the present invention is not interpreted to be limited by these Examples.

### [Example 1]

### <1-1> Cell culture

Adipocyte precursor cells capable of differentiating into brown adipocytes were isolated from mouse brown adipose tissue. Cells were cultured in Dulbecco's modified Eagle's medium (Welgene, LM001-07) containing 10% fetal bovine serum (FBS, Gibco, 16000044) and 1% Penicillin Streptomycin (Welgene, LS202-02) at 37°C in a humidified atmosphere with 5% CO₂. When the cells reached about 100% confluency, the cells were exposed to a differentiation medium supplemented with 2.5 mM isobutyl methylxanthine (IBMX, Cayman, I5879), 0.125 mM indomethacin (Cayman, 70270), 1 µM dexamethasone (Cayman), 1 µg/mL insulin (Sigma, I9278), and 1 nM triiodothyronine (T3, Cayman, 6028) for 3 days for adipogenesis. Then, the cells were maintained in a growth medium containing 1 µg/mL insulin and 1 nM triiodothyronine (T3) for 3 days.

### <1-2> Animals

6-week-old C57BL/6 male mice and CIDEA reporter mice were used according to the approved protocols by Institutional Animal Care and Use Committees of Seoul National University (SNU-201107-1, SNU-201221-3) for *in vivo* experiments. The mice were housed under a 12 h-light/12 h-dark cycle condition with free access to a normal chow diet (NCD, Purina Lab, 38057, protein: 24.52% calories, carbohydrate: 63.07% calories, and fat: 12.41% calories) and water at 22 ± 1°C. The mice were treated intraperitoneally with LJ-4378 (1 mg·kg-1day-1) dissolved in 0.2% DMSO (diluted in PBS) for 10 days.

For a diet-induced obesity model, mice were fed a high-fat diet (HFD)(Research Diets, D12492, protein: 20% kcal, carbohydrate: 20% kcal, and fat: 60% kcal) for 8 weeks. Then, LJ-4378 (1 mg·kg-1) was injected into the mice once daily for 10 days.

### [Example 2]

### Synthesis and profile of LJ-4378 and derivatives thereof

### <2-1> Synthesis of LJ-4378

D-mannose was converted into a glycosyl donor (compound 1) by [Non-Patent Document 1]. The glycosyl donor (compound 1) was condensed with 2-amino-6-chloropurine in the presence of TMSOTf as a Lewis acid to afford a β-anomer (compound 2, 30%) as a single stereoisomer (FIG. 1A). Anomeric assignment was easily achieved with a ¹H NMR experiment that showed a nuclear Overhauser effect between H-8 and 3'-H. Treatment of the 2-amino-6-chloro derivative (compound 2) with isoamyl nitrite, iodine, and methylene iodide in the presence of CuI afforded a 2-iodo-6-chloro derivative (compound 3), which was converted into a 2-iodo-6-amino derivative (compound 4) upon treatment with methanolic ammonia. A Sonogashira coupling reaction of compound 4 with 1-hexyne in the presence of bis(triphenylphosphine)palladium dichloride yielded a 2-hexynyl derivative (compound 5). Finally, removal of compound 5 with 1 N HCl synthesized a final 2-hexynyl-4'-thioadenosine derivative LJ-4378 (2-(6-amino-2-(hex-1-yn-1-yl)-9H-purin-9-yl)tetrahydrothiophene-3,4-diol, compound 6).

### <2-2> Synthesis of LJ-4378 derivatives

D-mannose was converted into a glycosyl donor (compound 1) by [Non-Patent Document 1]. The glycosyl donor (compound 1) was subjected to Vorbruggen condensation with silylated 2-iodo-6-chloropurine in the presence of TMSOTf as a Lewis acid to afford a β-anomer (compound 3, 60%) along with a trace amount of an α-anomer (FIG. 1B). The anomeric arrangement of compound 3 was confirmed by ¹H NMR NOE experiments. A strong NOE effect was observed between H-8 and 3'-H of compound 3, confirming the β arrangement, whereas the corresponding α-anomer showed no such NOE effect.

Subsequently, compound 3 was subjected to Sonogashira coupling reaction using propyne and butyne in the presence of tetrakis(triphenylphosphine)palladium and cesium carbonate to obtain a C2-propynyl derivative (compound 7a) and a C2-butynyl derivative (compound 7b), respectively. Compound 7a and compound 7b were treated with 1 N HCl to produce a 2-propynyl-6-chloro derivative (compound 8a) and a 2-butynyl-6-chloro derivative (compound 8b), respectively. The 2-propynyl-6-chloro derivative (compound 8a) was treated with tert-butanol and ammonia in 3-halobenzylamine to obtain a 2-propynyl-4'-thioadenosine derivative (compound 9a) and 2-propynyl-N6-3-halobenzyl-4'-thioadenosine derivatives (compounds 9b to 9e), respectively. Similarly, the 2-butynyl-6-chloro intermediate (compound 8b) was converted into a 2-butynyl-4'-thioadenosine derivative (compound 9f) and 2-butynyl-N6-3-halobenzyl-4'-thioadenosine derivatives (compounds 9g to 9j). The specific preparation methods are as follows: [Preparation Method 1] to [Preparation Method 12].

### [Preparation Method 1]

### (2R,3R,4S)-2-(6-chloro-2-(prop-1-yn-1-yl)-9H-purin-9-yl)tetrahydrothiophene-3,4-diol (compound 8a).

Tetrakis(triphenylphosphine)palladium (248 mg, 0.21 mmol), copper iodide (49 mg, 0.25 mmol), and cesium carbonate (700 mg, 2.14 mmol) were added to a stirred solution of compound 3 (943 mg, 2.14 mmol) in anhydrous N,N-dimethylformamide (25 mL). The stirred reaction mixture was bubbled with propyne gas at room temperature for 10 minutes and additionally stirred at room temperature for 1 hour. The reaction mixture was evaporated under reduced pressure, and a crude product compound 7a was applied to the next reaction without further purification. 1 N HCl (5 mL) was added to a stirred ice-cold solution of the crude product compound 7a in tetrahydrofuran (5 mL), and the mixture was stirred at room temperature for 15 hours. The reaction mixture was neutralized with a 1 N NaOH solution and carefully evaporated under reduced pressure. The residue was purified by silica gel column chromatography (methylene chloride/methanol = 24:1) to obtain compound 8a (0.301 g, 45% from compound 3) as a white solid: mp 101-103°C; UV (MeOH) λ ₘₐₓ284 nm; ¹H NMR (500 MHZ, CD ₃OD): *δ*8.85 (s, 1H), 6.08 (d, *J=* 6.6 Hz, 1H), 4.71-4.69 (m, 1H), 4.46 (q, *J=* 3.6 Hz, 1H), 3.55 (dd, *J=* 4.4, 11.0 Hz, 1H), 2.96 (dd, *J=* 3.4, 11.0 Hz, 1H), 2.11 (s, 3H); [a]²⁰_{D}= -45.0 *(c0.10,* MeOH); HRMS (FAB+): *m*/*z* calculated for C₁₂H₁₂ClN₄O₂S (M+H) ⁺311.0369, Found: 311.0374. Anal. Calcd for C₁₂H₁₂ClN₄O₂S: C, 46.38; H, 3.57; N, 18.03. Found: C, 46.78; H, 3.24; N, 18.04.

### [Preparation Method 2]

### (2R,3R,4S)-2-(2-(but-1-yn-1-yl)-6-chloro-9H-purin-9-yl)tetrahydrothiophene-3,4-diol (compound 8b).

Tetrakis(triphenylphosphine)palladium (429 mg, 0.37 mmol), copper iodide (85 mg, 0.44 mmol), and cesium carbonate (1.21 g, 3.71 mmol) were added to a stirred solution of compound 3 (1.63 g, 3.71 mmol) in anhydrous N,N-dimethylformamide (25 mL). The stirred reaction mixture was bubbled with butyne gas at room temperature for 10 minutes and additionally stirred at room temperature for 1 hour. The reaction mixture was evaporated under reduced pressure and used in the next reaction without further purification. 1 N HCl (5 mL) was added to a stirred ice-cold solution of the crude product compound 7b in tetrahydrofuran (5 mL), and the mixture was stirred at room temperature for 15 hours. The reaction mixture was neutralized with a 1 N NaOH solution and evaporated under reduced pressure. The residue was purified by silica gel column chromatography (methylene chloride/methanol = 24:1) to obtain compound 8b (530 mg, 44% from compound 3) as a white solid: mp 105-107°C; UV (MeOH) λ ₘₐₓ284 nm; ¹H NMR (500 MHz, CD ₃OD): *δ*8.86 (s, 1H), 6.08 (d, *J=* 6.5 Hz, 1H), 4.69 (m, 1H), 3.54 (dd, *J=* 4.4, 11.0 Hz, 1H), 3.30 (bs, 1H), 2.96 (dd, *J=* 3.5, 11.0 Hz, 1H), 2.50 (q, *J=* 7.5 Hz, 2H), 1.27 (t, *J=* 7.5 Hz, 3H); ¹³C NMR (125 MHz, CD ₃OD): *δ*154.2, 151.7, 148.7, 147.8, 132.6, 93.4, 81.6, 80.5, 75.1, 65.3, 36.1, 14.3, 14.2; [a]²⁰_{D} = -38.20 *(c0.10,* MeOH); HRMS (FAB+): *m*/*z* calculated for C₁₃H₁₄ClN₄O₂S (M+H) ⁺325.0526, Found: 325.0528. Anal. Calcd for C₁₃H₁₄ClN₄O₂S: C, 48.08; H, 4.03; N, 17.25. Found: C, 48.09; H, 4.23; N, 17.01.

### [Preparation Method 3]

### (2R,3R,4S)-2-(6-amino-2-(prop-1-yn-1-yl)-9H-purin-9-yl)tetrahydrothiophene-3,4-diol (compound 9a)

A solution of compound 8a (50 mg, 0.16 mmol) in NH₃/tBuOH (5 mL) was stirred in a steel bomb at 100°C for 12 hours. The reaction mixture was evaporated, and the residue was purified by silica gel column chromatography (methylene chloride/methanol = 24:1) to obtain 9a (45 mg, 85%) as a white solid: mp 247-249°C; UV (MeOH) λ ₘₐₓ271 nm; ¹H NMR (500 MHz, DMSO- *d* ₆): *δ*8.46 (s, 1H), 7.35 (bs, 2H), 5.84 (d, *J=* 7.2 Hz, 1H), 5.54 (d, *J=* 6.2 Hz, 1H), 5.35 (d, *J=* 4.1 Hz, 1H), 4.60 (dt, *J=* 3.2, 6.8 Hz, 1H), 4.34-4.32 (m, 1H), 3.40 (dd, *J=* 4.1, 10.8 Hz, 1H), 2.78 (dd, *J=* 2.8, 10.8 Hz, 1H), 2.01 (s, 3H); ¹³C NMR (125 MHz, DMSO- *d* ₆): *δ*155.4, 149.9, 145.3, 140.5, 118.3, 82.0, 80.2, 78.4, 72.1, 61.3, 34.3, 3.40; [a]²⁰_{D} = -38.0 (*c*0.10, MeOH); HRMS (FAB+): *m*/*z* calculated for C₁₂H₁₄N₅O₂S (M+H) ⁺292.0868, Found: 292.0867. Anal. Calcd for C₁₂H₁₃N₅O₂S: C, 49.47; H, 4.50; N, 24.04. Found: C, 49.29; H, 4.21; N, 23.98.

### * General procedure for synthesis of compounds 9b-e

Triethylamine (3 eq.) and 3-halobenzylamine (1.5 eq.) were added to a stirred solution of diol compound 8a (50 mg, 0.16 mmol) in ethanol (5 mL) at room temperature, and the mixture was stirred at room temperature for 24 hours. The solvent was evaporated for 48 hours, and the residue was purified by silica gel column chromatography (methylene chloride/methanol = 35:1) to obtain compounds 9b to 9e as white solids.

### [Preparation Method 4]

### (2R,3R,4S)-2-(6-((3-fluorobenzyl)amino)-2-(prop-1-yn-1-yl)-9H-purin-9-yl)tetrahydro thiophene-3,4-diol (compound 9b).

Yield: 81%; white solid; mp 237-239°C; UV (MeOH) λ ₘₐₓ275 nm; ¹H NMR (500 MHz, DMSO- *d* ₆): *δ*8.52-8.45 (m, 2H), 7.34 (q, *J=* 7.7 Hz, 1H), 7.15-7.03 (m, 3H), 5.86 (d, *J=* 7.2 Hz, 1H), 5.55 (d, *J=* 6.2 Hz, 1H), 5.36 (d, *J=* 4.0 Hz, 1H), 4.68-4.61 (m, 3H), 4.34 (bs, 1H), 3.39 (dd, *J=* 3.9, 10.7 Hz, 1H), 2.80 (dd, *J=* 2.6, 10.8 Hz, 1H), 2.01 (s, 3H); ¹³C NMR (125 MHz, DMSO- *d* ₆): *δ*163.1, 161.1, 153.9, 149.3, 145.5, 142.9, 140.6, 130.2, 122.9, 118.7, 113.5 (CF), 81.9, 80.7, 78.4, 72.1, 61.3, 42.3, 34.3, 3.4; [a]²⁰_{D} = -32.0 *(c0.10,* MeOH); HRMS (FAB+): *m*/*z* calculated for C₁₉H₁₉FN₅O₂S (M+H) ⁺400.1244, Found: 400.1249. Anal. Calcd for C₁₉H₁₈FN₅O₂S: C, 57.13; H, 4.54; N, 17.53. Found: C, 57.24; H, 4.94; N, 17.13.

### [Preparation Method 5]

### (2R,3R,4S)-2-(6-((3-chlorobenzyl)amino)-2-(prop-1-yn-1-yl)-9H-purin-9-yl)tetrahydro thiophene-3,4-diol (compound 9c).

Yield: 78%; white solid; mp 224-226°C; UV (MeOH) λ ₘₐₓ275 nm; ¹H NMR (500 MHz, DMSO- *d* ₆): *δ*8.51 (s, 1H), 8.47 (bs, 1H), 7.37-7.28 (m, 4H), 5.86 (d, *J=* 7.2 Hz, 1H), 5.54 (d, *J=* 6.2 Hz, 1H), 5.34 (d, *J=* 4.0 Hz, 1H), 4.67 (bs, 2H), 4.61 (s, 1H), 4.34 (s, 1H), 3.40 (dd, *J=* 3.9, 10.7 Hz, 1H), 2.80 (dd, J= 2.6, 10.8 Hz, 1H), 2.02 (s, 3H); ¹³C NMR (125 MHz, DMSO- *d* ₆): *δ*153.9, 149.3, 145.5, 142.4, 140.6, 132.8, 130.1, 126.8, 126.5, 125.6, 118.7, 81.9, 80.7, 78.4, 72.1, 61.3, 42.2, 34.3, 3.47; [a]²⁰_{D} = -42.0 (*c*0.10, MeOH); HRMS (FAB+): *m*/ zcalculated for C₁₉H₁₉ClN₅O₂S (M+H) ⁺416.0948, Found: 416.0952. Anal. Calcd for C₁₉H₁₈ClN₅O₂S: C, 54.87; H, 4.36; N, 16.84. Found: C, 54.76; H, 4.46; N, 16.45.

### [Preparation Method 6]

### (2R,3R,4S)-2-(6-((3-bromobenzyl)amino)-2-(prop-1-yn-1-yl)-9H-purin-9-yl)tetrahydro thiophene-3,4-diol (compound 9d).

Yield: 79%; white solid; mp 240-242°C; UV (MeOH) λ ₘₐₓ275 nm; ¹H NMR (500 MHz, DMSO- *d* ₆): *δ*8.51 (s, 1H), 8.47 (bs, 1H), 7.52 (s, 1H), 7.42 (d, *J=* 7.6 Hz, 1H) 7.32-7.25 (m, 2H), 5.86 (d, *J=* 7.2 Hz, 1H), 5.53 (d, *J=* 6.2 Hz, 1H), 5.35 (d, *J=* 4.0 Hz, 1H), 4.67 (bs, 2H), 4.61 (bs, 1H), 4.34 (s, 1H), 3.40 (dd, *J=* 3.9, 10.7 Hz, 1H), 2.80 (dd, *J=* 2.6, 10.8 Hz, 1H), 2.02 (s, 3H); ¹³C NMR (125 MHz, DMSO- *d* ₆): *δ*153.9, 149.3, 145.5, 142.7, 140.6, 130.4, 129.7, 129.5, 126.1, 121.5, 118.7, 81.9, 80.7, 78.4, 72.1, 61.3, 42.1, 34.3, 3.45; [a]²⁰_{D} = 1.40 ( *c*0.10, MeOH); HRMS (FAB+): *m*/*z* calculated for C₁₉H₁₉BrN₅O₂S (M+H) ⁺462.0424, Found: 462.0446. Anal. Calcd for C₁₉H₁₈BrN₅O₂S: C, 49.57; H, 3.94; N, 15.21. Found: C, 49.17; H, 4.12; N, 15.43.

### [Preparation Method 7]

### (2R,3R,4S)-2-(6-((3-iodobenzyl)amino)-2-(prop-1-yn-1-yl)-9H-purin-9-yl)tetrahydro thiophene-3,4-diol (compound 9e).

Yield: 77%; white solid; mp 253-255°C; UV (MeOH) λ ₘₐₓ275 nm; ¹H NMR (500 MHz, DMSO- *d* ₆): *δ*8.50 (s, 1H), 8.45 (bs, 1H), 7.71 (s, 1H), 7.58 (d, *J=* 7.2 Hz, 1H), 7.33 (d, *J=* 7.6 Hz, 1H), 7.11 (t, *J=* 7.7 Hz, 1H), 5.86 (d, *J=* 7.1 Hz, 1H), 5.54 (d, *J=* 6.1 Hz, 1H), 5.34 (d, *J=* 3.8 Hz, 1H), 4.63 (bs, 3H), 4.34 (s, 1H), 3.41 (dd, *J=* 3.8, 10.6 Hz, 1H), 2.80 (dd, *J=* 2.6, 10.8 Hz, 1H), 2.02 (s, 3H); ¹³C NMR (125 MHz, DMSO- *d* ₆): *δ*153.9, 149.3, 145.5, 142.5, 140.6, 135.7, 135.4, 130.4, 126.5, 118.7, 94.7, 81.9, 80.7, 78.4, 72.1, 61.3, 40.0, 34.3, 3.4; [a]²⁰_{D} = 107.0 (*c*0.10, MeOH); HRMS (FAB+): *m*/*z* calculated for C₁₉H₁₉IN₅O₂S (M+H) ⁺508.0304, Found: 508.0312. Anal. Calcd for C₁₉H₁₈IN₅O₂S: C, 44.98; H, 3.58; N, 13.80. Found: C, 45.13; H, 3.18; N, 14.12.

### [Preparation Method 8]

### (2R,3R,4S)-2-(6-amino-2-(but-1-yn-1-yl)-9H-purin-9-yl)tetrahydrothiophene-3,4-diol (compound 9f)

Compound 8b (50 mg, 0.15 mmol) was converted into compound 9f (40 mg, 84%) as a white solid following the same procedure used to prepare compound 9a: mp 202-204°C; UV (MeOH) λ ₘₐₓ272 nm; ¹H NMR (500 MHz, DMSO- *d* ₆): *δ*8.47 (s, 1H), 7.36 (s, 1H), 5.86 (d, *J=* 7.2 Hz, 1H), 5.55 (d, *J=* 5.2 Hz, 1H), 5.37 (bs, 1H), 4.59-4.60 (m, 1H), 4.34 (bs, 1H), 3.41-3.35 (m, 2H; merged with water peak), 2.79 (dd, *J=* 2.4, 10.0 Hz, 1H), 2.40 (q, *J=* 7.5 Hz, 2H), 1.15 (t, *J=* 7.5 Hz, 3H); ¹³C NMR (125 MHz, DMSO- *d* ₆): *δ*155.6, 149.9, 145.7, 140.4, 118.2, 86.7, 80.6, 78.5, 72.1, 61.1, 34.3, 13.3, 11.9; [a]²⁰_{D} = 90.00 (*c*0.12, MeOH); HRMS (FAB+): *m*/*z* calculated for C₁₃H₁₆N₅O₂S (M+H) ⁺306.1025, Found: 306.1011. Anal. Calcd for C₁₃H₁₅N₅O₂S: C, 51.13; H, 4.95; N, 22.94. Found: C, 51.42; H, 4.55; N, 23.10.

### * General procedure for synthesis of compounds 9g to 9j.

Triethylamine (3 eq.) and 3-halobenzylamine (1.5 eq.) were added to a stirred solution of diol compound 8b (50 mg, 0.15 mmol) in ethanol (5 mL) at room temperature, and the mixture was stirred at room temperature for 24 hours. The solvent was evaporated for 48 hours, and the residue was purified by silica gel column chromatography (methylene chloride/methanol = 20:1) to obtain compounds 9g to 9j as white solids.

### [Preparation Method 9]

### (2R,3R,4S)-2-(2-(but-1-yn-1-yl)-6-((3-fluorobenzyl)amino)-9H-purin-9-yl)tetrahydrothiophene-3,4-diol (compound 9g)

Yield: 80%; white solid; mp 230-231°C; UV (MeOH) λ ₘₐₓ275 nm; ¹H NMR (500 MHz, DMSO- *d* ₆): *δ*8.52 (s, 1H), 8.46 (bs, 1H), 7.35 (q, *J=* 7.7 Hz, 1H), 7.16-7.11 (m, 2H), 7.06-7.02 (m, 1H), 5.88 (d, *J=* 7.3 Hz, 1H), 5.55 (d, *J=* 6.2 Hz, 1H), 5.36 (d, *J=* 3.8 Hz, 1H), 4.69 (bs, 2H), 4.60 (bs, 1H), 4.34 (s, 1H), 3.41 (dd, *J=* 3.9, 10.7 Hz, 1H), 2.80 (dd, *J=* 2.6, 10.8 Hz, 1H), 2.40 (q, *J*= 7.5 Hz, 2H), 1.15 (t, *J*= 7.5 Hz, 3H); ¹³C NMR (125 MHz, DMSO-*d* ₆): *δ*163.1, 161.2, 153.9, 149.4, 145.6, 142.9, 140.5, 130.1, 123.0, 118.6, 113.5 (CF), 86.9, 80.9, 78.5, 72.1, 61.1, 42.3, 34.3, 13.2, 11.9; [a]²⁰_{D} = -15.10 *(c0.10,* MeOH); HRMS (FAB+): *m*/*z* calculated for C₂₀H₂₁FN₅O₂S (M+H) ⁺414.1400, Found: 414.1396. Anal. Calcd for C₂₀H₂₀FN₅O₂S: C, 58.10; H, 4.88; N, 16.94. Found: C, 57.99; H, 5.12; N, 16.44.

### [Preparation Method 10]

### (2R,3R,4S)-2-(2-(but-1-yn-1-yl)-6-((3-chlorobenzyl)amino)-9H-purin-9-yl)tetrahydrothiophene-3,4-diol (compound 9h).

Yield: 78%; white solid; mp 236-238°C; UV (MeOH) λ ₘₐₓ275 nm; ¹H NMR (500 MHz, DMSO- *d* ₆): *δ*8.52 (s, 1H), 8.47 (bs, 1H), 7.38-7.27 (m, 4H), 5.88 (d, *J=* 7.2 Hz, 1H), 5.56 (d, *J=* 6.1 Hz, 1H), 5.37 (d, *J=* 3.5 Hz, 1H), 4.68 (bs, 2H), 4.60 (s, 1H), 4.34 (bs, 1H), 3.41 (dd, *J=* 3.8, 10.6 Hz, 1H), 2.80 (dd, *J=* 2.4, 10.6 Hz, 1H), 2.40 (q, *J=* 7.5 Hz, 2H), 1.15 (t, *J=* 7.5 Hz, 3H); ¹³C NMR (125 MHz, DMSO- *d* ₆): *δ*153.9, 149.4, 145.6, 142.4, 140.5, 132.8, 130.1, 126.9, 126.6, 125.7, 118.6, 86.9, 80.8, 78.5, 72.1, 61.1, 42.2, 34.3, 13.2, 11.9; [a]²⁰_{D} = -28.0 (*c*0.10, MeOH); HRMS (FAB+): *m*/*z* calculated for C₂₀H₂₁ClN₅O₂S (M+H) ⁺430.1104, Found: 430.1111. Anal. Calcd for C₂₀H₂₀ClN₅O₂S: C, 55.88; H, 4.69; N, 16.29. Found: C, 55.54 H, 4.25; N, 16.29.

### [Preparation Method 11]

### (2R,3R,4S)-2-(6-((3-bromobenzyl)amino)-2-(but-1-yn-1-yl)-9H-purin-9-yl)tetrahydrothiophene-3,4-diol (compound 9i).

Yield: 77%; white solid; mp 222-224°C; UV (MeOH) λ ₘₐₓ275 nm; ¹H NMR (500 MHz, DMSO- *d* ₆): *δ*8.52 (s, 1H), 8.48 (bs, 1H), 7.53 (s, 1H), 7.42 (d, *J=* 7.6 Hz, 1H), 7.33-7.25 (m, 2H), 5.87 (d, *J=* 5.2 Hz, 1H), 5.54 (d, *J=* 5.2 Hz, 1H), 5.36 (bs, 1H), 4.67 (bs, 1H), 4.60 (bs, 2H), 4.34 (bs, 1H), 3.40 (dd, *J=* 3.9, 10.7 Hz, 1H), 2.80 (dd, *J=* 2.5, 10.7 Hz, 1H), 2.41 (q, *J=* 7.4 Hz, 2H), 1.16 (t, *J=* 7.5 Hz, 3H); ¹³C NMR (125 MHz, DMSO- *d* ₆): *δ*153.9, 149.4, 145.6, 142.7, 140.5, 130.4, 129.8, 129.5, 126.1, 121.5, 118.6, 87.0, 80.8, 78.5, 72.1, 61.1, 42.2, 34.3, 13.2, 11.9; [a]²⁰_{D} = -9.90 (*c*0.10, MeOH); HRMS (FAB+): *m*/*z* calculated for C₂₀H₂₁BrN₅O₂S (M+H) ⁺476.0580, Found: 476.0594. Anal. Calcd for C₂₀H₂₀BrN₅O₂S: C, 50.64; H, 4.25; N, 14.76. Found: C, 50.76; H, 4.32; N, 14.98.

### [Preparation Method 12]

### (2R,3R,4S)-2-(2-(but-1-yn-1-yl)-6-((3-iodobenzyl)amino)-9H-purin-9-yl)tetrahydrothiophene-3,4-diol (compound 9j).

Yield: 77%; white solid; mp 239-241°C; UV (MeOH) λ ₘₐₓ275 nm; ¹H NMR (500 MHz, DMSO- *d* ₆): *δ*8.52 (s, 1H), 8.45 (bs, 1H), 7.72 (s, 1H), 7.58 (d, *J=* 7.6 Hz, 1H), 7.33 (d, *J=* 7.3 Hz, 1H), 7.11 (t, *J=* 7.7 Hz, 1H), 5.88 (d, *J=* 7.3 Hz, 1H), 5.55 (d, *J=* 6.1 Hz, 1H), 5.36 (d, *J=* 3.9 Hz, 1H), 4.63-4.60 (m, 3H), 4.30 (bs, 1H), 3.41 (dd, *J=* 3.9, 10.7 Hz, 1H), 2.81 (dd, *J=* 2.7, 10.8 Hz, 1H), 2.41 (q, *J=* 7.4 Hz, 2H), 1.16 (t, *J=* 7.5 Hz, 3H); ¹³C NMR (125 MHz, DMSO-*d* ₆): *δ*153.8, 149.3, 145.6, 142.5, 140.5, 135.8, 135.3, 130.4, 126.5, 118.6, 94.6, 86.9, 80.8, 78.5, 72.1, 61.1, 42.1, 34.3, 13.2, 11.9; [a]²⁰_{D} = -13.60 (*c*0.10, MeOH); HRMS (FAB+): *m*/*z* calculated for C₂₀H₂₁IN₅O₂S (M+H) ⁺522.0461, Found: 522.0468. Anal. Calcd for C₂₀H₂₀IN₅O₂S: C, 46.07; H, 3.87; N, 13.43. Found: C, 46.23; H, 3.47; N, 13.95.

### <2-1> Profile of LJ-4378 and derivatives thereof

LJ-4378 is a material with excellent binding ability to A_{2A}AR and A_{3A}R, and showed very high subtype selectivity. Furthermore, as a result of evaluating the cAMP activation ability of the most important A_{2A}AR, LJ-4378 showed EC₅₀ and efficacy similar to those of a full agonist CGS21680. In the case of A_{3A}R, as a result of performing experiments using an A₃AR full agonist Cl-IB-MECA, LJ-4378 at high concentrations acted as a full antagonist capable of completely inhibiting cAMP production, and the K_{B} value showed high efficacy at the nM level (FIG. 1C). That is, LJ-4378, which strongly acts as an agonist and an antagonist on A_{2A}AR and A_{3A}R, respectively, was able to effectively increase cAMP production.

The binding affinity of LJ-4378 and derivatives thereof to A_{2A}AR and A₃AR is as shown in the following [Table 1]. All binding affinity experiments were performed using adherent HEK293 cells stably transfected with cDNAs encoding appropriate hAR bonds, and were performed using 1 nM [3H]17, 10 nM [3H]18, or 0.2 nM [3H]16 as radioligands for A_{2A} and A_{3A}R, respectively. Values were normalized to a non-specific binder 5'-N-ethylcarboxamidoadenosine (NECA, 10 µM).

**[Table 1]**

| **Compound** | R¹ | R² | Kᵢ(hA₂AR), nM | Kᵢ(hA₃AR), nM |
|---|---|---|---|---|
| 9a | H | Me | 117 | 64.7 |
| 9b | 3-F-Bn | Me | 772 | 30.5 |
| 9c | 3-Cl-Bn | Me | 221 | 1.5 |
| 9d | 3-Br-Bn | Me | 469 | 11.1 |
| 9e | 3-I-Bn | Me | 121 | 20.1 |
| 9f | H | Et | 23.9 | 67.3 |
| 9g | 3-F-Bn | Et | 914 | 9.5 |
| 9h | 3-Cl-Bn | Et | 239 | 3.8 |
| 9i | 3-Br-Bn | Et | 113 | 0.293 |
| 9j | 3-I-Bn | Et | 58.0 | 8.6 |
| 6(LJ-4378) | H | nBu | 7.19 | 11.8 |

### [Example 3]

### Dose-dependent effects of LJ-4378 on lipolysis and mitochondrial content

### <3-1> Cytotoxicity

LJ-4378 is a dual-acting ligand with A_{2A}AR agonist and A₃AR antagonist activity. Cell viability was evaluated according to the manufacturer's instructions using EZ-CYTOX (DoGEN, EZ-3000). Absorbance was measured at 450 nm using a microplate reader (Thermo MLTLTISKAN GO, 8816-2015).

Cytotoxicity analysis of LJ-4378 showed that concentrations less than 100 µM had no effect on cell viability in brown adipocytes (FIG. 2(A)).

### <3-2> Lipolytic effects of LJ-4378

Since A_{2A}AR increases cAMP levels and activates protein kinase A signaling in adipocytes, the lipolytic effects of LJ-4378 were investigated in a dose-response manner.

**[Table 2]**

| **Antibody** | **Host** | **Company** | **Catalog** # | **Dilution** |
|---|---|---|---|---|
| UCP1 | Rabbit | Alpha diagnostic | UCP11-A | 1:1000 |
| COXIV | Rabbit | Cell signaling | 4850 | 1:1000 |
| HSL | Rabbit | Cell signaling | 4107 | 1:1000 |
| Phospho-HSL | Rabbit | Cell signaling | 45804 | 1:1000 |
| CREB | Rabbit | Cell signaling | 9197 | 1:1000 |
| P-CREB | Rabbit | Cell signaling | 9198 | 1:1000 |
| Tubulin | Rabbit | Cell signaling | 2148 | 1:1000 |
| MCAD | Mouse | Santa Cruz | sc-365030 | 1:1000 |
| Total oxphos | Mouse | Cell signaling | 110413 | 1:1000 |

In the case of Western blots, the primary antibodies used for Western blots are summarized in [Table 2] above. An anti-rabbit horseradish peroxidase antibody (1:3000, Thermo Fisher, 31460) and an anti-mouse horseradish peroxidase antibody (1:3000, Jackson, 115-035-174) were used as secondary antibodies. All antibodies were diluted in a blocking buffer (5% skimmed milk or 5% bovine serum albumin in TBST). Western blot images were acquired using a Fusion Solo chemiluminescence imaging system (Vilber Lourmat) and analyzed with Evolution Capt software (version 17.03). NIH ImageJ software was used for quantification.

The free fatty acid (FFA) levels in the medium were measured using an NEFA reagent (WAKO, 436-91693) according to the manufacturer's product protocol.

Immunoblot analysis showed that LJ-4378 upregulated the expression level of phosphorylated hormone-sensitive lipase (P-HSL) from serine 660 in a dose-dependent manner (FIG. 2(C): (10⁻¹¹ M) 1.55±0.11-fold; (10⁻⁹ M) 3.03±0.21-fold, (10⁻⁷) 6.64±0.34-fold, (10⁻⁵) 6.87±0.69-fold increases). Consistently, the levels of free fatty acid (FFA) were also increased by LJ-4378 (FIG. 2(D): 1.92 ± 0.03-fold increase).

Furthermore, mitochondrial proteins increased according to the dosage of LJ-4378 (FIG. 2(E): ATP synthase alpha-subunit (ATP5A): (10⁻¹¹ M) 2.20±0.08-fold, (10⁻⁹ M) 3.51±0.05-fold, (10⁻⁷) 3.94±0.06, (10⁻⁵) 3.72±0.04-fold increases, ubiquinol-cytochrome c reductase core protein 2 (UQCRC2): (10⁻¹¹ M) 2.15±0.03-fold, (10⁻⁹ M) 3.96±0. (10⁻⁷) 4.21±0.13-fold, (10⁻⁵) 4.02±0.08-fold increases, succinate dehydrogenase complex iron sulfur subunit B (SDHB): (10⁻¹¹ M) 1.87±0.28-fold, (10⁻⁹ M) 3.47±0.18-fold, (10⁻⁷) 3.41±0.47-fold, (10⁻⁵) 3.71±0.25-fold increases, MCAD: (10⁻¹¹ M) 1.53±0.03-fold, (10⁻⁹M) 1.79±0.03-fold (10⁻⁷) 1.79±0.09-fold, (10⁻⁵) 2.13±0.03-fold increases). Based on these dose-response evaluations, a concentration of 0.1 µM was used in subsequent experiments.

### [Example 4]

### Comparison of LJ-4378 with A_{2A}AR agonist or A₃AR antagonist

### <4-1> Confirmation of adenosine receptor expression levels

qPCR analysis was performed to investigate the adenosine receptor expression levels in brown adipocytes differentiated from immortalized brown adipocyte precursor cells. Primers used for the qPCR analysis are listed in the following [Table 3].

**[Table 3]**

| **Gene** | **Forward (5'→3')** | **Reverse (5'→3')** |
|---|---|---|
| *Ucpl* | TGG CCT CTC AGT GGA TGT G (SEQ ID NO: 1) | CGT GGT CTC CCA GCA TAG AAG |
| | | (SEQ ID NO: 2) |
| *Cidea* | TGC TCT TCT GTA TCG CCC AGT (SEQ ID NO: 3) | GCC GTG TTA AGG AAT CTG CTG |
| | | (SEQ ID NO: 4) |
| *Ppia* | GTG GTC TTT GGG AAG GTG AA(SEQ ID NO: 5) | TTA CAG GAC ATT GCG AGC AG |
| | | (SEQ ID NO: 6) |
| *Adora1* | TGGCTCAGTTCGTGCATCAT (SEQ ID NO: 7) | ATCCTTACCCATCGACTGCC (SEQ ID NO: 8) |
| *Adora2a* | CAGAGAAGGGAAGCAATCGCA (SEQ ID NO: 9) | ACCATGACTTCTACAGGGGGT (SEQ ID NO: 10) |
| *Adora2b* | GCGTCCCGCTCAGGTATAAA (SEQ ID NO: 11) | CAATGCCAAAGGCAAGGACC (SEQ ID NO: 12) |
| *Adora3* | AGT AAG AAC GGT GGC CCT CT (SEQ ID NO: 13) | ATC CCT GCC TTC CCA TTA ACC |
| | | (SEQ ID NO: 14) |

The A_{2A}AR showed the highest expression (FIG. 3A: 343.7±68.04), and the A_{3A}R (FIG. 3A: 0.19±0.03) was the second highest among the adenosine receptors investigated.

### <4-2> Comparison of lipolytic effects of LJ-4378, A_{2A}AR agonist, or A₃AR antagonist

The lipolytic effects of LJ-4378 were compared with those of CGS21680 (Cayman, 124431-80-7), which are an A_{2A}AR agonist and an A₃AR antagonist, and LJ-4433, respectively.

Glycerol and free fatty acid (FFA) levels in a medium were measured using a glycerol reagent (Sigma Aldrich, F6428) and a NEFA reagent (WAKO, 436-91693) according to the manufacturer's product protocols.

In the analysis of both glycerol and FFA, LJ-4378 treatment showed the greatest fold change of 1.61±0.07 and 1.59±0.1 compared to CGS21680 (FIG. 3B: 1.51±0.08-fold; FIG. 3C: 1.29±0.09-fold) and LJ-4433 (FIG. 3B: 1.22±0.07-fold; FIG. 3C: 0.92±0.09-fold), respectively (FIGS. 3B and 3C). Immunoblot analysis further confirmed the additive effect of LJ-4378, as shown by upregulated levels of PKA downstream proteins, including phosphorylated cAMP response element binding protein (P-CREB)(2.32±0.05-fold increase by LJ-4378; 2.00±0.11-fold increase by CGS21680; 1.40±0.11-fold increase by LJ-4433) and P-HSL (2.10±0.12-fold increase by LJ-4378, 1.54±0.05-fold increase by CGS21680, 1.22±0.07-fold increase by LJ-4433)(FIG. 3D).

### [Example 5]

### Effects of LJ-4378 on brown adipocytes

The effects of LJ-4378 on mitochondrial content and metabolic activity capacity in brown adipocytes were evaluated.

Oxygen consumption rate (OCR) was measured using an XFp analyzer. Cells were cultured in XF DMEM basal medium (Agilent, 103575-100, pH 7.4) supplemented with 4 mM L-glutamine (Sigma, G8540) and 25 mM D-glucose (Sigma, G7021) at 37°C. The XFp Cell Mitochondrial Stress Test Kit (Agilent, 103010-100) was prepared and calculated sequentially with optimal final concentrations of 2.5 µM oligomycin, 0.5 µM FCCP and 0.5 µM rotenone/antimycin A and basal, maximal and proton leakage. Then, the OCR was normalized to protein concentration.

The cells were exposed to MitoTracker^{™} Red CMXRos (1:3000, Invitrogen, M7512) at 37°C for 15 minutes to stain mitochondria, and then fixed in 4% paraformaldehyde (PFA, Sigma, 158127). Images were acquired with an LSM800 confocal microscope (Zeiss, Germany) and analyzed with Zen software (version 3.0).

The expression levels of a brown adipocyte marker UCP1 and a mitochondrial protein COXIV were significantly increased by LJ-4378 treatment by 2.93±0.23-fold and 2.18±0.15-fold, respectively (FIG. 4A). MitoTracker staining also showed that LJ-4378 increased mitochondrial membrane potential by 2.12±0.06-fold in brown adipocytes (FIG. 4B). In addition, the present inventors compared the effects of LJ-4378 on mitochondrial content with those of an A_{2A}AR agonist (CGS21680) and an A₃AR antagonist (LJ-4433). Both CGS21680 and LJ-4433 increased UCP1, COXIV, and MCAD (FIG. 4D). However, larger effects were observed in LJ-4378 (FIG. 4D: 2.67±0.38-fold increase in UCP1, 2.53±0.08-fold increase in COXIV, and 4.91±0.02-fold increase in MCAD), suggesting an additive effect.

As a result of investigating oxygen consumption rate for functional analysis, it was found that LJ-4378 increased the OCR related to basal (30.5%), maximal (49.1%), and ATP production (29.3%) (FIG. 4C).

### [Example 6]

### Effects of LJ-4378 on mitochondrial activity and in vivo energy expenditure in adipose tissue

*Ex vivo* electron transport activity associated with mitochondrial oxidative phosphorylation was evaluated by monitoring the reduction of 0.1% triphenyl tetrazolium chloride (TTC, Sigma, T8877).

Indirect calorimetry was performed using a PhenoMaster (TSE Systems) to measure expenditure (EE), VO₂, VCO₂, activity, and food intake. Body components were measured by nuclear magnetic resonance scanning Echo MRI-700 (Echo Medical Systems).

In *in situ* staining with triphenyltetrazolium chloride (TTC), a redox indicator showed that LJ-4378 upregulated mitochondrial activity in all adipose tissue depots (FIG. 5A: 2.86±0.24-fold increase in BAT; 2.07±0.17-fold increase in iWAT; 1.72±0.16-fold increase in gWAT).

The indirect calorimetric analysis showed that LJ-4378 treatment increased oxygen consumption by 17.8% to increase energy expenditure by 17.0%, while activity and food intake were unaffected (FIG. 5B).

### [Example 7]

### Effects of LJ-4378 on adipose tissue browning in vivo

The synergistic effects of LJ-4378 on adipose tissue browning were investigated in CIDEA reporter mice, which reflect the expression of CIDEA, a marker of brown adipocytes, through fluorescent and luminescent signals.

*In vivo* bioluminescence was detected using an optical imaging device (Ami-X, Spectral Instruments Imaging). To detect *in vivo* bioluminescence signals, D-luciferin (150 mgkg-1, Goldbio) was injected intraperitoneally into mice. Aura Software (Spectral Instruments Imaging, Version 2.2.1.1) was used for quantification. Furthermore, *ex vivo* imaging tissues were collected from the CIDEA reporter mice treated with D-luciferin (150 mgkg-1, Goldbio, i.p.). During imaging, isolated tissues were maintained in 12-well plates containing D-luciferin in PBS (300 µg/ml).

LJ-4378 significantly increased the bioluminescence intensity of BAT and iWAT (FIGS. 6A and 6B). In particular, iWAT was significantly increased compared to CGS21680 and LJ-4433, indicating an additive effect of LJ-4378 on browning, particularly, in white adipose tissue. According to *in vivo* bioluminescence results, LJ-4378 treatment caused the most significant increase in the *ex vivo* luminescence signals of BAT and iWAT (FIG. 6C).

The mRNAlevels of brown adipocyte markers were also investigated, and as a result, the investigation showed a consistent result that LJ-4378 caused the greatest increase in gene expression associated with browning, including *Ucp1* (2.13±0.21-fold increase by LJ-4378, 1.72±0.17-fold increase by CGS21680, and 1.74±0.08-fold increase by LJ-4433)and *Cidea* (1.72±0.06-fold increase by LJ-4378, 1.29±0.12-fold increase by CGS21680, 1.12±0.04-fold increase by LJ-4433, and increase by LJ-4433).

### [Example 8]

### Anti-obesity effects of LJ-4378

It was investigated whether the lipolysis and browning effects of LJ-4378 have anti-obesity effects.

Mice fed a HFD were used as a diet-induced obesity model. Body weight changes were monitored during treatment, and fat and body components of the mice were measured after treatment.

For histological analysis, adipose tissue was fixed in 10% formalin and then embedded in a paraffin block. Paraffin sections were stained with hematoxylin/eosin (H&E).

For a glucose tolerance test (GTT), 20% D-glucose (1 gkg-1, Sigma Aldrich, G7021) was injected intraperitoneally into mice. A glucose meter (Gluco Dr. Top, allmedicus, AGM-4100, Anyang, Republic of Korea) was used to measure blood glucose concentrations.

LJ-4378-treated HFD-fed mice showed 8.24% and 24.18% reduction in body weight and fat mass, respectively, (FIG. 7A and 7B). In addition, the weights of iWAT and gWAT were significantly decreased by LJ-4378 treatment in HFD-fed mice (FIG. 7C: 30.1% reduction in iWAT, and 18.6% reduction in gWAT). The reduced adipocyte size in iWAT and gWAT was further supported by H&E staining (FIGS. 7D and 7E). Furthermore, LJ-4378 improved glucose tolerance in HFD-fed mice (FIG. 7F).

Next, the present inventors investigated the expression levels of mitochondrial proteins and PKA downstream markers to determine whether such anti-obesity and antidiabetic effects were induced by increased mitochondrial content and activation of PKA signaling. As a result, LJ-4378 upregulated COXIV (BAT: 5.91±0.23-fold, iWAT: 1.24±0.04-fold), UCP1 (BAT: 1.33±0.06-fold, iWAT: 3.34±0.36-fold), P-CREB (BAT: 1.54±0.03-fold; iWAT: 3.86±0.12-fold), and P-HSL (BAT: 2.31±0.18-fold, iWAT: 1.72±0.04-fold) in BAT and iWAT of diet-fed and obese mice (FIG. 8).

Serving in a dual mode as agonists for A_{2A}AR and antagonists for A_{3A}R, the ligand LJ-4378 and derivatives thereof that act on the adenosine receptors according to the present invention significantly increased mitochondrial proteins, UCP1, and COXIV and exhibited excellent anti-obesity effects in high-fat diet mice. Therefore, the ligand acting on adenosine receptors according to the present invention can be effectively used in a composition for the prevention, alleviation, or treatment of obesity, and in a therapeutic method and preparation of a drug for the same.

### [Data and statistical analysis]

Prism 7 software (GraphPad Software, USA) was used for statistical analysis. Data were shown as mean ± SEM. An unpaired t-test was used between two groups to measure statistical significance.

### [Industrial Applicability]

Serving in a dual mode as agonists for A_{2A}AR and antagonists for A₃AR, the ligand LJ-4378 and derivatives thereof that act on the adenosine receptors according to the present invention significantly increased mitochondrial proteins, UCP1, and COXIV and exhibited excellent anti-obesity effects in high-fat diet mice. Therefore, the ligand acting on adenosine receptors according to the present invention can be effectively used as a composition for the prevention, alleviation, or treatment of obesity, and thus has industrial applicability.

### [Sequence Listing Free Text]

SEQ ID NO: 1 represents the forward primer used for qPCR analysis for Ucp1.
SEQ ID NO: 2 represents the reverse primer used for qPCR analysis for Ucp1.
SEQ ID NO: 3 represents the forward primer used for qPCR analysis for Cidea.
SEQ ID NO: 4 represents the reverse primer used for qPCR analysis for Cidea.
SEQ ID NO: 5 represents the forward primer used for qPCR analysis for Ppia.
SEQ ID NO: 6 represents the reverse primer used for qPCR analysis for Ppia.
SEQ ID NO: 7 represents the forward primer used for qPCR analysis for Adora1.
SEQ ID NO: 8 represents the reverse primer used for qPCR analysis for Adora1.
SEQ ID NO: 9 represents the forward primer used for qPCR analysis for Adora2a.
SEQ ID NO: 10 represents the reverse primer used for qPCR analysis for Adora2a.
SEQ ID NO: 11 represents the forward primer used for qPCR analysis for Adora2b.
SEQ ID NO: 12 represents the reverse primer used for qPCR analysis for Adora2b.
SEQ ID NO: 13 represents the forward primer used for qPCR analysis for Adora3.
SEQ ID NO: 14 represents the reverse primer used for qPCR analysis for Adora3.

## Claims

1. A pharmaceutical composition for the prevention or treatment of a disease associated with A_{2A} adenosine receptor (AR) and/or A₃ adenosine receptor (AR), comprising a compound represented by the following [Chemical Formula 1], an isomer thereof, or a pharmaceutically acceptable salt thereof: 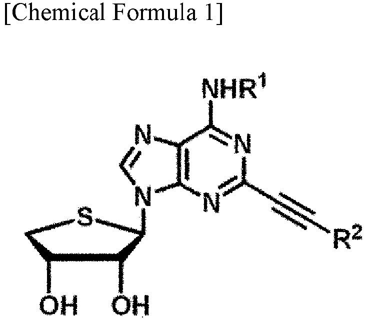 wherein R1 is H, a C1-C10 alkyl group, or an aryl-C1-C10 alkyl group, and
R2 is a C1-C10 alkyl group.

2. The pharmaceutical composition of claim 1, wherein R1 is H, a methyl group, or an aryl-C1-C10 alkyl group, and
R2 is a C1-C4 alkyl group.

3. The pharmaceutical composition of claim 1, wherein the compound represented by [Chemical Formula 1], the isomer thereof, or the pharmaceutically acceptable salt thereof is any one or more selected from the group consisting of
(1) 2-(6-amino-2-(hex-1-yn-1-yl)-9H-purin-9-yl)tetrahydrothiophene-3,4-diol;
(2) (2R,3R,4S)-2-(6-amino-2-(prop-1-yn-1-yl)-9H-purin-9-yl)tetrahydrothiophene-3,4-diol;
(3) (2R,3R,4S)-2-(6-((3-fluorobenzyl)amino)-2-(prop-1-yn-1-yl)-9H-purin-9-yl)tetrahydrothiophene-3,4-diol;
(4) (2R,3R,4S)-2-(6-((3-chlorobenzyl)amino)-2-(prop-1-yn-1-yl)-9H-purin-9-yl)tetrahydrothiophene-3,4-diol;
(5) (2R,3R,4S)-2-(6-((3-bromobenzyl)amino)-2-(prop-1-yn-1-yl)-9H-purin-9-yl)tetrahydrothiophene-3,4-diol;
(6) (2R,3R,4S)-2-(6-((3-iodobenzyl)amino)-2-(prop-1-yn-1-yl)-9H-purin-9-yl)tetrahydrothiophene-3,4-diol;
(7) (2R,3R,4S)-2-(6-amino-2-(but-1-yn-1-yl)-9H-purin-9-yl)tetrahydrothiophene-3,4-diol;
(8) (2R,3R,4S)-2-(2-(but-1-yn-1-yl)-6-((3-fluorobenzyl)amino)-9H-purin-9-yl)tetrahydrothiophene-3,4-diol;
(9) (2R,3R,4S)-2-(2-(but-1-yn-1-yl)-6-((3-chlorobenzyl)amino)-9H-purin-9-yl)tetrahydrothiophene-3,4-diol;
(10) (2R,3R,4S)-2-(6-((3-bromobenzyl)amino)-2-(but-1-yn-1-yl)-9H-purin-9-yl)tetrahydrothiophene-3,4-diol; and
(11) (2R,3R,4S)-2-(2-(but-1-yn-1-yl)-6-((3-iodobenzyl)amino)-9H-purin-9-yl)tetrahydrothiophene-3,4-diol.

4. The pharmaceutical composition of claim 1, wherein the compound represented by [Chemical Formula 1], the isomer thereof, or the pharmaceutically acceptable salt thereof is an agonist for A_{2A} adenosine receptor (AR) and an antagonist for A₃ adenosine receptor (AR).

5. The pharmaceutical composition of claim 1, wherein the disease associated with A_{2A} adenosine receptor (AR) and/or A₃ adenosine receptor (AR) is obesity.

6. A health functional food composition for the prevention or alleviation of obesity, comprising a compound represented by the following [Chemical Formula 1], an isomer thereof, or a pharmaceutically acceptable salt thereof: 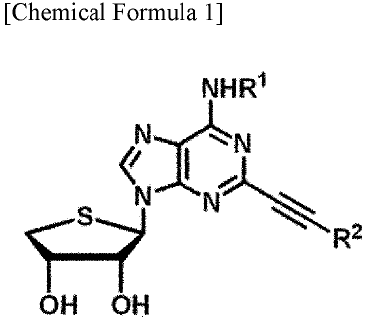 wherein R1 is H, a C1-C10 alkyl group, or an aryl-C1-C10 alkyl group, and
R2 is a C1-C10 alkyl group.

7. A method for treating a disease associated with A_{2A} adenosine receptor (AR) and/or A₃ adenosine receptor (AR), the method comprising administering a compound represented by the following [Chemical Formula 1], an isomer thereof, or a pharmaceutically acceptable salt thereof to a patient with a disease associated with A_{2A} adenosine receptor (AR) and/or A₃ adenosine receptor (AR): 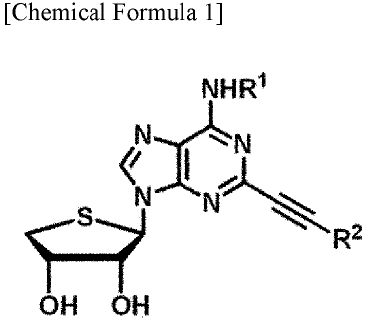 wherein R1 is H, a C1-C10 alkyl group, or an aryl-C1-C10 alkyl group, and
R2 is a C1-C10 alkyl group.

8. A use of a compound represented by the following [Chemical Formula 1], an isomer thereof, or a pharmaceutically acceptable salt thereof in preparing a drug for the treatment of a disease associated with A_{2A} adenosine receptor (AR) and/or A₃ adenosine receptor (AR): 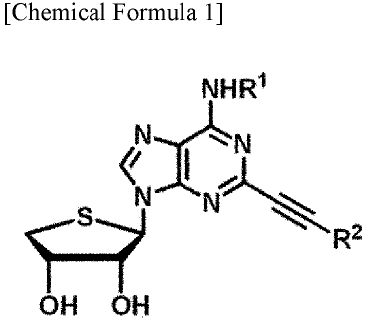 wherein R1 is H, a C1-C10 alkyl group, or an aryl-C1-C10 alkyl group, and
R2 is a C1-C10 alkyl group.
